# EUROPEAN PATENT APPLICATION

(11) **EP 4 342 975 A1**
(43) Date of publication of application: **27.03.2024**
(21) Application number: 23162143.4
(22) Date of filing: 15.03.2023
(51) Int. Cl.: C12M 3/00, C12M 1/00, C12M 1/36

(54) **METHOD FOR PERFORMING BIOPROCESSES ON CELL CULTURES**

(30) Priority: 21.09.2022 WO PCT/IB2022/000491; 13.12.2022 WO PCT/EP2022/085651; 05.01.2023 EP 23150494
(71) Applicant: The Automation Partnership (Cambridge) Ltd., Hertfordshire SG8 5WY (GB)
(72) Inventor: Huttlestone, Daren Stephen, Royston, Herts SG8 9TF (GB); Stacey, Adrian, Cambridge, CB4 1FY (GB); Prouté, Fanny, Cambridge, CB4 1FY (GB)
(74) Representative: Gottschald Patentanwälte Partnerschaft mbB

(57) **Abstract**

Proposed is a method for performing bioprocesses on cell cultures, wherein the bioprocesses are being performed on an integrated bioprocessing system (1), wherein while performing the bioprocesses the integrated bioprocessing system (1) transfers a medium used in the bioprocess, in particular the cell cultures, through tubes, wherein the integrated bioprocessing system (1) comprises a tube connection system (2) for connecting tubes of the integrated bioprocessing system (1) as needed by welding, wherein a first tube (4), a first tube holder (7) and a first fluidic structure (12) for receiving and/or providing the medium are carried by a movable first carrier (10), wherein the first tube (4) is held by the first tube holder (7) and is fluidically connected to the first fluidic structure (12), wherein the first carrier (10) and with the first carrier (10) the first tube (4), the first tube holder (7) and the first fluidic structure (12) are transported to a welding location within the integrated bioprocessing system (1), wherein in a welding routine at the welding location the tube connection system (2) connects the first tube (4) and a second tube (5) such that a tube connection (3) is formed, wherein the integrated bioprocessing system (1) transfers the medium from or to the first fluidic structure (12) of the first carrier (10) through the tube connection (3) after welding.

## Description

The invention relates to a method for performing bioprocesses on cell cultures according to claim 1, to an integrated bioprocessing system for performing bioprocesses on cell cultures according to claim 7, to an integrated bioprocessing system for performing bioprocesses on cell cultures according to claim 17, to a sealing unit according to claim 18 and to a device for transporting a tube according to claim 19.

The term "bioprocess" comprises processes in the broad field of biotechnology, exemplarily in the area of cell and gene therapy including allogenic or autologous production of genetically modified immune cells. In bioprocesses, living cell cultures are taken from a patient, generated, modified and/or used, particularly to obtain a specific desired product. The desired product may be the cells themselves and/or a product that is produced by the cells. For example, if a respective product shall be obtained, a certain cell culture might be generated and modified such that the product is produced and obtained thereafter. Generally, different kinds of cell cultures might lead to different kinds of products. In the context of this application, the term "bioprocess" has to be understood broadly such that it does comprise partial processes, like generation or modification of cell cultures or usage of cell cultures to obtain the product, as well as entire processes on cell cultures. An example for a typical bioprocess is the manufacturing of autologous T cells that are modified to express a chimeric antigen receptor (CAR). These cells might be used for the treatment of various types of hematologic malignancies, including different types of leukemia (blood cancer). Other cell therapies based on naive cells, in particular stem cells and their derivates, are also of interest.

For bioprocesses involving the use of cell cultures, particularly for cell and gene therapy, process flexibility is especially relevant, since operations of the bioprocesses has to be adjusted and individually performed depending on different circumstances. For that reason, nowadays, such bioprocesses are often performed in integrated bioprocessing systems, which are flexible and automatized to a certain level.

An example for an integrated bioprocessing system for performing bioprocesses is disclosed in WO 2021/212124 A1. Here, a method for the modular and parallelized processing of cell cultures on an integrated bioprocessing system is disclosed.

Another integrated bioprocessing system is known from WO 2023/281257 A1. In this integrated bioprocessing system, tubes are held by holders and welded by a tube welding system. Generally, movement of components like the tubes and the tube welding system is performed by a robotic mechanism. Finding and handling flexible tubes is very challenging. Controlling a tip of a robotic manipulator and detecting the position of a tube with sufficiently low tolerances to grab the tube needs very sophisticated controlling and sensorial capacities. There is therefore a need to reduce the degrees of freedom of the integrated bioprocessing system and in particular a need to find a way of either completely removing a robotic manipulator or at least increasing the tolerances that the tip of the robotic manipulator may have with regard to the position of the tube such that the robotic manipulator can reliably grab the tube.

During the performance of bioprocesses different kinds of operations may be performed, which thus require different kinds of media, such as the cell cultures themselves or other fluids, particularly liquids, such as culture media for cell expansion, activation reagents, viral vector compositions, washing buffers, magnetic bead solutions etc. Since, when performing a bioprocess by using an integrated bioprocessing system, operations of the bioprocesses might be performed at different locations within the integrated bioprocessing system or with different components of the integrated bioprocessing system, transferring of the respective medium or media is mandatory. Therefore, known integrated bioprocessing systems often comprise tubes, which are linked such that transferring of the medium or media is enabled. The linked tubes form tube connections.

Even if methods for connecting different components with tubes are generally well established in the art, the tube connections within the known integrated bioprocessing systems are either pre-linked, for example by utilizing tube-sets, or manually linked by an operator, for example before or during a bioprocess to be performed. While pre-linked tube connections, however, limit the flexibility of the integrated bioprocessing systems, since the transfer of the medium or media is pre-determined to a certain degree, manually linked tube connections increase the risk of contamination of the cell cultures. For that reason, known methods for performing bioprocesses on cell cultures by using an integrated bioprocessing system are either limited regarding flexibility of the performance of the bioprocesses themselves or regarding sterility providence.

The present invention thus is based on the problem of providing a method for performing bioprocesses on cell cultures by using an integrated bioprocessing system, which is improved regarding flexibility without compromising the sterility of the cell cultures.

The above-noted object is solved by the features of claim 1.

The main realization of the present invention is that using welding to connect tubes on an integrated bioprocessing system is an easy way of creating any number of tube connections for transferring media in a flexible way through the integrated bioprocessing system. It becomes possible to automate the transfer of different media, in particular the cell cultures, from and to different containers, apparatus, cartridges or receptacles during the bioprocess. Sealed fluidic structures can be created and connected as necessary. Accompanying, the formed tube connections by welding are sterile such that the risk of any contamination of the cell cultures is reduced to a minimum, particularly if the welding routine is performed automatically. Furthermore, using a carrier for carrying tubes and using a holder for holding tubes enables higher automatization of the overall bioprocess due to simpler handling of the tubes within the integrated bioprocessing system.

In comparison to the known robotic welding mechanism, the present application presents various ways of handling the location of a tube by means other than a robotic manipulator to reduce the degrees of freedom of the integrated bioprocessing system. It is possible to still use a robotic manipulator, such as a robotic mechanism, for welding, but even then, as the tubes are also handled by other means, the control of the robotic manipulator would be easier. In some embodiments, no robotic manipulator is used for the tube welding.

Proposed is a method for performing bioprocesses on cell cultures, wherein the bioprocesses are being performed on an integrated bioprocessing system, wherein while performing the bioprocesses the integrated bioprocessing system transfers a medium used in the bioprocess, in particular the cell cultures, through tubes, wherein the integrated bioprocessing system comprises a tube connection system for connecting tubes of the integrated bioprocessing system as needed by welding, wherein a first tube, a first tube holder, and a first fluidic structure for receiving and/or providing the medium are carried by a movable first carrier, wherein the first tube is held by the first tube holder and is fluidically connected to the first fluidic structure, wherein the first carrier and with the first carrier the first tube, the first tube holder and the first fluidic structure are transported to a welding location within the integrated bioprocessing system, wherein in a welding routine at the welding location the tube connection system connects the first tube and a second tube such that a tube connection is formed, wherein the integrated bioprocessing system transfers the medium from or to the first fluidic structure of the first carrier through the tube connection after welding.

According to an advantageous embodiment of claim 2, the welding routine comprises different phases to be performed, namely an arrangement phase, a trimming phase and a welding phase. Each phase by itself or a combination of these phases offers a simple sequence, which leads to a, in particular sterile, tube connection of the first tube and the second tube, when comprised by the welding routine.

Claim 3 further specifies the trimming phase as well as the welding phase. During an advantageous heating step of the trimming phase, a blade is heated by a blade heater of a tube cutting unit to a certain temperature. Here, on the one hand, the blade itself is sterilized such that contamination can be avoided, when the tubes are being cut. On the other hand, the heated blade may also enable smoothed cutting of the tubes, since these are often made out of plastic material, which melds at certain temperatures, when being cut. During an advantageous alignment step of the trimming phase, the tube cutting unit is aligned such that the tube cutting unit is positioned relatively to the first tube and/or the second tube, wherein the positioning may ensure proper cutting of the tubes. During an advantageous cutting step of the trimming phase, the first tube and/or the second tube are being cut by the tube cutting unit. During an advantageous contacting step of the welding phase, the tubes might be axially aligned to enable a proper tube connection, when the tubes are welded. Alternatively or additionally during the contacting step, the tube cutting unit might be at least partly moved such that the tubes can be easily brought in contact, wherein movement of the tube cutting unit enables the tubes to be contacted. Alternatively or additionally during the contacting step, the tubes might be brought in contact by axial movement of at least one of the tubes, wherein the tubes are merged together, in particular if the material of the tubes is still heated because of the cutting process. In particular, if the tubes were already axially aligned, movement in merely axial direction of the tubes protects from improper tube connections.

The advantageous embodiments according to claim 4 is directed to a disconnection routine, which makes it possible to disconnect the tubes, if required. The disconnection routine is performed by a tube disconnection arrangement. The disconnection routine may comprise a sealing phase, wherein at least one tube is being sealed such that the fluidical connection between the tubes is interrupted. The disconnection routine may comprise a disconnecting phase, wherein the tubes are being cut such that the physical connection between the tubes is interrupted.

According to a preferred embodiment of claim 5, the method comprises performing of an integrity testing routine. Here, the integrity of the tube connection, which were formed by welding, can be tested such that if the integrity of the tube connection is not verified, an emergency routine for preserving the cell culture can be initiated. The emergency routine might comprise securing the sterility of the tubes and/or giving a notification to a user and/or initiating a disconnection routine and/or initiating another welding routine.

According to claim 6, the first tube can be connected to a third tube such that a further tube connection is formed in a welding routine. The advantageous embodiment enables to connect the first tube to different tubes as required such that the provided method provides further flexibility.

According to a further aspect, the carrier, which carries the first tube holder and the first fluidic structure and the first tube, is inserted into the integrated bioprocessing system and/or leave the integrated bioprocessing system while the carrier carries the first tube holder and the first fluidic structure and the first tube and while the first tube holder holds the first tube. This allows for preconfiguring the carriers with the fluidic structures. In particular, the carrier enters and leaves the integrated bioprocessing system with the fluidic structure without the fluidic structure being reconfigured in the integrated bioprocessing system, apart from the tube welding.

According to a further aspect, the first tube is put into the first tube holder outside of the integrated bioprocessing system, in particular manually, prior to the first tube holder entering the integrated bioprocessing system. Entering and/or leaving the integrated bioprocessing system may be defined by an enclosure of the integrated bioprocessing system which may comprise one or more defined interfaces for carriers entering and/or leaving the integrated bioprocessing system.

A second teaching according to claim 7, which is of equal importance, relates to an integrated bioprocessing system for performing bioprocesses on cell cultures, wherein while performing the bioprocesses a medium used in the bioprocess, in particular the cell cultures, is transferable by the integrated bioprocessing system through tubes. The integrated bioprocessing system comprises a first tube for transferring medium and a second tube for transferring medium, wherein the first tube is held by a first tube holder, and is fluidically connected to a first fluidic structure for receiving and/or providing the medium, and a movable first carrier, wherein the first tube, the first tube holder and the first fluidic structure are carried by the first carrier and wherein the first carrier and with the first carrier the first tube, the first tube holder and the first fluidic structure are transportable to a welding location within the integrated bioprocessing system, and a tube connection system for connecting the first tube and the second tube in a welding routine at the welding location such that a tube connection is formed by welding, wherein the medium is transferable through the tube connection from or to the first fluidic structure of the first carrier after welding.

With the proposed integrated bioprocessing system, the proposed method can be performed and thus all features and explanations given with regard to the proposed method are fully applicable to the proposed integrated bioprocessing system and vice versa.

Claim 8 defines an advantageous embodiment, which enables simple positioning as well as simple handling of the second tube within the integrated bioprocessing system.

Claim 9 specifies the tube connection system of the integrated bioprocessing system. The tube connection system comprises a tube arrangement unit for arranging, in an arrangement phase of the welding routine, the first tube and the second tube relatively to each other in at least in an axial direction of the first tube and/or the second tube, such that the first tube and the second tube can be cut, and a tube cutting unit for cutting the first tube and the second tube in a trimming phase of the welding routine, wherein the tube arrangement unit is designed such that, in a welding phase of the welding routine, the first tube and the second tube are brought in contact and merged together such that the tube connection is formed.

According to claim 10, the tube arrangement unit may comprise the first and the second tube holder. The tube holders enable handling of the tubes, in particular during the welding routine. The tube arrangement unit may further comprise a first and/or second holder movement arrangement, which enable to move the first tube holder and/or the second tube holder respectively such that the first tube and/or the second tube can be moved accordingly in an indirect way, in particular for arrangement, alignment, handling or the like. Advantageously, the first holder movement arrangement comprises a first holder guide, which is particularly designed as a first rail unit, and/or a first holder drive unit. Alternatively or additionally, the second holder movement arrangement comprises a second holder guide, which is particularly designed as a second rail unit, and/or a second holder drive unit. The first holder guide and/or the second holder guide enable precise and, in particular, space-saving predefined movement of the first tube holder and/or the second tube holder. The first drive unit and/or the second drive unit enable driving the movement of the, especially railbounded, first tube holder and/or second tube holder. In particular, the combination of the respective holder guide and the drive unit may lead to a price-effective, highly precise and in particular space-saving movement system for moving the respective tube holder.

Claim 11 specifies a further option for moving the first tube and/or the second tube. While the foregoing embodiments may primarily enable indirect movement of the tubes by moving the tube holders holding the tubes, the advantageous embodiments according to claim 10 enable to move the first tube and/or the second tube in a direct way. This may lead to further degrees of movement such that the first tube and/or the second tube can be moved in at least a further direction. The tube arrangement unit may comprise a first tube drive unit and/or a second tube drive unit, which drives the movement of the respective tube.

In claim 12, the first drive unit and the second drive unit are further advantageously specified. With a respective drive roller, a constructively simple but equally effective option for moving the respective tube, in particular in an axial direction of the tube, can be provided. With a respective drive locker, the drive roller can be locked such that further movement of the respective tube, in particular in the axial direction of the tube, is avoided. With a respective passive roller, the respective tube can be positioned in the drive unit such that it is contacted by the drive roller. With a pretension arrangement, the grip of the drive roller on the tube can be improved by pretensioning the tube against the drive roller or vice versa.

For higher flexibility regarding the relative movement of the tubes and the tube cutting unit, the tube connection system advantageously comprise a tube connection head according to claim 13. The first tube holder and/or the second tube holder may be designed separately from the tube connection head. Here, the tube connection head may comprise the tube cutting unit such that the tube cutting unit can be moved relatively to the first tube holder and/or second tube holder and thus relatively to the first tube held by the first tube holder and/or the second tube held by the second tube holder. In a preferred embodiment, the tube connection head comprises a first tube receiver and/or a second tube receiver. Here, the first tube receiver and/or the second tube receiver might be designed as grippers of the tube connection head such that the first tube and/or the second tube might be gripped by the first tube receiver and/or the second tube receiver. Alternatively, the first tube receiver and/or the second tube receiver may be designed as tube holders with one or several features of the first tube holder and the second tube holder. The tube cutting unit might be part of the tube connection head or might be designed separately, wherein in both cases the tubes can be relatively moved against the tube cutting unit by the tube connection head.

According to the embodiments of claim 14, the first tube and/or the second tube, in particular when moved, can be guided. Since, tubes are often flexible, a first guiding arrangement and/or a second guiding arrangement may prevent bending of the first tube and/or the second tube and thus lead to a more precise movement of the respective tube. Advantageously, the first guiding arrangement comprises a first funnel and/or the second guiding arrangement comprises a second funnel, wherein the respective funnel provides a constructively easy, but reliable and robust guiding option of the respective tube. Due to the respective funnel, the guided tube may be guided along its axis.

The advantageous embodiment according to claim 15 concerns the position determination within the tube connection system. In particular, because of the highly flexible movement of the tubes, the tube holders and/or the tube connection head, in particular because of the carrying carrier or carriers, a position determination arrangement can lead to higher precision accuracy when the tube connection system operates. The position determination arrangement may comprise a position sensor or several position sensors, which are commonly known in the art in wide variety for countless applications, so that these can be utilized at relatively cost-effective development costs.

According to an embodiment of claim 16, the tube connection system comprises a robotic mechanism. The robotic mechanism carries the tube connection head such that it can be moved in at least one direction. According to another embodiment of claim 16, the tube connection system comprises additionally or alternatively a conveyor mechanism, wherein the first carrier and/or the second carrier is movable by the conveyor mechanism. For example, if the first carrier, the first tube, the first tube holder and the first fluidic structure are part of a cartridge, with which a unit operation might be performed, and the second carrier, the second tube, the second tube holder and the second fluidic structure are part of a receptacle, with which a cell culture is transported, the conveyor mechanism makes it possible to move the cartridge and/or the receptacle directly. The respective tube, the respective tube holder and the respective fluidic structure are moved indirectly, since they are carried by the respective carrier. Especially the combination of the robotic mechanism and the conveyor mechanism can lead to a highly effective tube connection system, in particular for usage in an integrated bioprocessing system.

A third teaching according to claim 17, which is of equal importance, relates to an integrated bioprocessing system for performing bioprocesses on cell cultures, wherein while performing the bioprocesses a medium used in the bioprocess, in particular the cell culture, is transferable by the integrated bioprocessing system through tubes. The integrated bioprocessing system comprises a first tube for transferring medium and a second tube for transferring medium, wherein the first tube and the second tube are connected by a tube connection and are located at a disconnection location within the integrated bioprocessing system, wherein the first tube is held by a first tube holder, and is fluidically connected to a first fluidic structure for receiving and/or providing the medium via the first tube, and a movable first carrier, wherein the first tube, the first tube holder and the first fluidic structure are carried by the first carrier, and a tube disconnection arrangement for disconnecting the first tube and the second tube in a disconnection routine at the disconnection location such that the first tube and the second tube are fluidically and/or physically disconnected, wherein the first carrier and with the first carrier the first tube, the first tube holder and the first fluidic structure are transportable from the disconnection location after disconnection of the first tube and the second tube.

With the proposed integrated bioprocessing system, the proposed method can be performed and thus all features and explanations given with regard to the proposed method are fully applicable to the proposed integrated bioprocessing system according to claim 17 and vice versa. Furthermore, all features and explanations given with regard to the proposed integrated bioprocessing system according to claim 7 are fully applicable to the proposed integrated bioprocessing system according to claim 17 and vice versa.

A fourth teaching, which is of equal importance, relates to a tube disconnection arrangement for disconnecting a first tube and a second tube of an integrated bioprocessing system in a disconnection routine such that the first tube and the second tube are fluidically and/or physically disconnected, wherein the tube disconnection arrangement comprises a sealing unit for sealing the first tube and/or the second tube in a sealing phase such that a first sealing and/or a second sealing is or are formed, wherein the sealing unit comprises at least one sealing surface, which is designed to crimp the first tube and/or the second tube along the axial direction of the respective tube such that the first tube and/or the second tube is or are self-sealed.

The proposed tube disconnection arrangement may be used in the proposed method and thus all features and explanations given with regard to the proposed method are fully applicable to the proposed tube disconnection arrangement and vice versa. The proposed tube disconnection arrangement may be used in the proposed integrated bioprocessing system according to claim 7 and/or in the proposed integrated bioprocessing system according to claim 17 and thus all features given with regard to the integrated bioprocessing systems are fully applicable to the proposed tube disconnection arrangement and vice versa.

A fifth teaching according to claim 18, which is of equal importance, relates to a sealing unit, in particular of a tube disconnection arrangement in an integrated bioprocessing system, for sealing a first tube and/or a second tube in a sealing phase such that a sealing is formed, wherein the sealing unit comprises at least one sealing surface, which is designed to crimp the first tube and/or the second tube along the axial direction of the respective tube such that the first tube and/or the second tube is or are self-sealed.

The proposed sealing unit may be used in the proposed method and thus all features and explanations given with regard to the proposed method are fully applicable to the proposed sealing unit and vice versa. The proposed sealing unit may be used in the proposed tube disconnection arrangement, the integrated bioprocessing system according to claim 7 and/or in the proposed integrated bioprocessing system according to claim 17 and thus all features given are fully applicable to the proposed sealing unit and vice versa.

A sixth teaching according to claim 19, which is of equal importance, relates to a device for transporting a tube, in particular within an integrated bioprocessing system, wherein the device comprises a carrier, a tube, a tube holder and a fluidic structure, wherein the carrier carries the tube , the tube holder and the fluidic structure such that the tube, the tube holder and the fluidic structure are transportable with the carrier, and, wherein the tube holder holds the tube in a defined position..

The proposed device is related to the proposed method, the proposed integrated bioprocessing system according to claim 7, the proposed integrated bioprocessing system according to claim 17, the proposed tube disconnection arrangement and the proposed sealing unit according to claim 18. Therefore, all features and explanations given are fully applicable to the proposed tube holder and vice versa.

Proposed according to a seventh teaching, which is of equal importance, is a method for performing bioprocesses on cell cultures, wherein the bioprocesses are being performed on an integrated bioprocessing system, wherein while performing the bioprocesses the integrated bioprocessing system transfers a medium used in the bioprocess, in particular the cell cultures, through tubes, wherein the integrated bioprocessing system comprises a tube connection system for connecting tubes of the integrated bioprocessing system as needed by welding, wherein a first tube, a first tube holder and a first fluidic structure for receiving and/or providing the medium are part of the integrated bioprocessing system, wherein the first tube is held by the first tube holder and is fluidically connected to the first fluidic structure, wherein the first tube holder is positioned at the welding location by a positioning mechanism separate of the tube connection system, wherein in a welding routine at the welding location the tube connection system connects the first tube and a second tube such that a tube connection is formed, wherein the integrated bioprocessing system transfers the medium from or to the first fluidic structure of the first carrier through the tube connection after welding.

All explanations given with regard to any other teaching may apply and vice versa.

If the tube is positioned, in particular only roughly, at the welding location, in particular at a predefined welding location used for many welding routines, the tube connection system may interact with the tubes by aiming, in particular only more or less, at the known welding location, which may be defined relative to the integrated bioprocessing system irrespective of the actual position of the tube, and the tube connection system may be positioned at the welding location in a repeatable process.

In one embodiment, the first carrier is positioned at the weld location, thereby positioning the first holder at the welding location. In another embodiment, then, the first holder is further repositioned such that it is positioned at the welding location more precisely. In another embodiment, only then, the tube connection system interacts with the tube. In another embodiment, only then, the tube connection system further repositions the tube, in particular by repositioning the tube holder or without repositioning the tube holder, for welding.

The tube connection system may over time interact with several, possibly tens or hundreds of, tube holders at the same welding location as the carriers and tube holders may change frequently as described.

According to one embodiment, the positioning mechanism may position the tube holder only in one or two dimensions, in particular dimensions parallel to the floor, at a time or at all. If the first tube holder is positioned along the gravity dimension, preferably, the positioning mechanism comprises separate mechanism for positioning the first tube holder along the gravity dimension and orthogonal thereto. Preferably, the positioning mechanism is configured to move the first tube holder in not more than two dimensions, preferably not more than one dimension, at the same time. Exemplarily, the positioning mechanism may be a conveyor moving the carrier or a rail unit moving the tube holder.

Proposed according to an eight teaching, which is of equal importance, is a method for performing bioprocesses on cell cultures, wherein the bioprocesses are being performed on an integrated bioprocessing system, wherein while performing the bioprocesses the integrated bioprocessing system transfers a medium used in the bioprocess, in particular the cell cultures, through tubes, wherein the integrated bioprocessing system comprises a tube connection system for connecting tubes of the integrated bioprocessing system as needed by welding, wherein a first tube, a first tube holder and a first fluidic structure for receiving and/or providing the medium are part of the integrated bioprocessing system, wherein the first tube is held by the first tube holder and is fluidically connected to the first fluidic structure, wherein the first tube holder fluidically closes, in particular pinches, the first tube, in particular reversibly, wherein in a welding routine at the welding location the tube connection system connects the first tube and a second tube such that a tube connection is formed, wherein the integrated bioprocessing system transfers the medium from or to the first fluidic structure of the first carrier through the tube connection after welding. The tube holder can be used for a further function in this way by providing extra security against incorrect welding. The closing may be released after the weld has been verified. If the weld is not verified, a user may for example remove the fluidic structure from the integrated bioprocessing system while the tube is still closed.

All explanations given with regard to any other teaching may apply and vice versa.

Proposed according to a ninth teaching, which is of equal importance, is a method for performing bioprocesses on cell cultures, wherein the bioprocesses are being performed on an integrated bioprocessing system, wherein while performing the bioprocesses the integrated bioprocessing system transfers a medium used in the bioprocess, in particular the cell cultures, through tubes, wherein the integrated bioprocessing system comprises a tube connection system for connecting tubes of the integrated bioprocessing system as needed by welding, wherein a first tube, a first tube holder and a first fluidic structure for receiving and/or providing the medium are part of the integrated bioprocessing system, wherein the first tube is held by the first tube holder and is fluidically connected to the first fluidic structure, wherein the first tube holder extrudes the first tube into the tube connection system, in particular prior to any interaction between the first tube and the tube connection system, wherein in a welding routine at the welding location the tube connection system connects the first tube and a second tube such that a tube connection is formed, wherein the integrated bioprocessing system transfers the medium from or to the first fluidic structure of the first carrier through the tube connection after welding. This is a further way of handling the tubes by means other than the tube connection system to reduce the complexity of the tube connection system.

All explanations given with regard to any other teaching may apply and vice versa.

Proposed according to a tenth teaching, which is of equal importance, is a method for performing bioprocesses on cell cultures, wherein the bioprocesses are being performed on an integrated bioprocessing system, wherein while performing the bioprocesses the integrated bioprocessing system transfers a medium used in the bioprocess, in particular the cell cultures, through tubes, wherein the integrated bioprocessing system comprises a tube connection system for connecting tubes of the integrated bioprocessing system as needed by welding, wherein a first tube, a first tube holder and a first fluidic structure for receiving and/or providing the medium are part of the integrated bioprocessing system, wherein the first tube is held by the first tube holder and is fluidically connected to the first fluidic structure, wherein the first tube holder holds the first tube in a predefined angle or angle range defined with respect to the integrated bioprocessing system as such and/or defined with respect to the tube connection system and/or defined with respect to the second tube prior to any interaction between the first tube and the tube connection system, wherein in a welding routine at the welding location the tube connection system connects the first tube and a second tube such that a tube connection is formed, wherein the integrated bioprocessing system transfers the medium from or to the first fluidic structure of the first carrier through the tube connection after welding. If an angle or angle range and therefore at least to some extent a direction of the tube is defined, the tube connection system can more easily interact with the tube. It may even be the case that the tube connection system cannot be turned orthogonally to the ground or not turned at all such that a predefined orientation of the tubes is necessary. For example, moving the tube connection system in cartesian directions into multiple predefined welding locations without turning the tube connection system simplifies the control of the tube connection system greatly.

All explanations given with regard to any other teaching may apply and vice versa.

An eleventh teaching is directed to a method of welding, in particular sealing, a tube at least partially along an extension of the tube in particular in an integrated bioprocessing system.

All explanations given with regard to any other teaching may apply and vice versa.

All features described herein can be combined into further teachings and all features of all teachings can be combined. Any devices, components, method steps and the like described are preferred embodiments in any combinations. In particular, the carrier is not an essential feature in all embodiments of the invention. Other ways of improving the handling of tubes and the tube welding as described herein are equally preferred.

In the following, embodiments of the invention are explained with respect to the drawing. The drawing shows in
- Fig. 1: a schematic representation of a provided integrated bioprocessing system with an augmented representation of a tube connection,
- Fig. 2: in a) to e) a schematic representation of a welding routine in top view,
- Fig. 3: in a) to d) a schematic representation of a provided tube connection system in the welding routine according to Fig. 2a) to 2e),
- Fig. 4: in a) to d) a schematic representation of a disconnection routine in top view,
- Fig. 5: an embodiment of a provided tube holder in top view with an augmented representation of the tube holder in sectional view and with a first guiding arrangement and a second guiding arrangement arranged at the tube holder,
- Fig. 6: a further embodiment of a provided tube connection system,
- Fig. 7: a schematic representation of an integrity testing routine,
- Fig. 8: in a) and b) a schematic representation of a provided tube disconnection arrangement in a disconnection routine, in particular during a sealing phase,
- Fig. 9: an embodiment of a provided tube connection system with a first and second guiding arrangement as a part of a tube connection head.

Provided is a method for performing bioprocesses on cell cultures. The bioprocesses being performed may be equal, but in particular different and/or individual bioprocesses. However, each bioprocess may comprise at least a unit operation, namely a basic step, which is performed to reach a certain physical or chemical effect. The cell cultures, on which the bioprocesses are performed, might be of the same or different kind. Preferably, the bioprocesses might be performed at least partly simultaneously.

Here and preferably, the cell cultures are liquid immune or naive cell cultures.

The term "liquid immune cell culture" is to be understood in a broad sense and refers to an immune cell culture comprising at least one type of immune cells suspended as particles in any type of liquid. As will be explained below, the liquid immune cell culture may comprise other cell types that are not immune cells. Hence, the term "liquid immune cell culture" refers to a liquid immune cell culture at any stage during the bioprocess. Consequently, the type and fraction of immune cells present in the liquid immune cell culture will change during the bioprocess applied as certain immune cells are enriched or depleted from the liquid immune cell culture and/or the immune cells are genetically modified.

The term "immune cells" generally refers to different types of white blood cells. Hence, the term "immune cells" includes a variety of cells, for example, but not limited to dendritic cells, T lymphocytes, also referred to as T cells, B lymphocytes, natural killer cells, macrophages or the like. Immune cells may also include subtypes of immune cells, for example tumor-infiltrating lymphocytes or different types of T cells. Subtypes of a certain type of immune cells may be classified based on the type of antigen present at the cell surface. Hence, the term immune cells may for example refer to T cells comprising the surface antigen CD4 ("CD4+ T cells"). Typically, a certain type of immune cells, e.g., T cells, preferably a certain subtype of immune cells, e.g., CD4+ T cells, will be selectively enriched by the bioprocess, while other immune cells, e. g. macrophages, and/or other cell types that are not immune cells, e. g., erythrocytes, and/or other subtypes of immune cells, e.g., CD8+ T cells, will be depleted from the liquid immune cell culture. The immune cells to be enriched are referred to as target immune cells, all other components to be depleted from the liquid immune cell culture are referred to "impurities". Further, and as mentioned above, the target immune cells might be genetically modified.

The term "naive cells" refers to cells that can still differentiate into different target cell types. In particular, stem cells and their derivatives prior to full differentiation into a specific cell type are naive cells. The term also comprises naive immune cells.

The term "liquid" is to be understood in a broad sense as well and refers to any liquid and/or particle-containing liquid that is processed within the integrated bioprocessing system. Hence, the term liquid might refer to media, waste, the liquid immune cell culture, byproducts obtained during the bioprocess, samples and/or an initial immune cell culture.

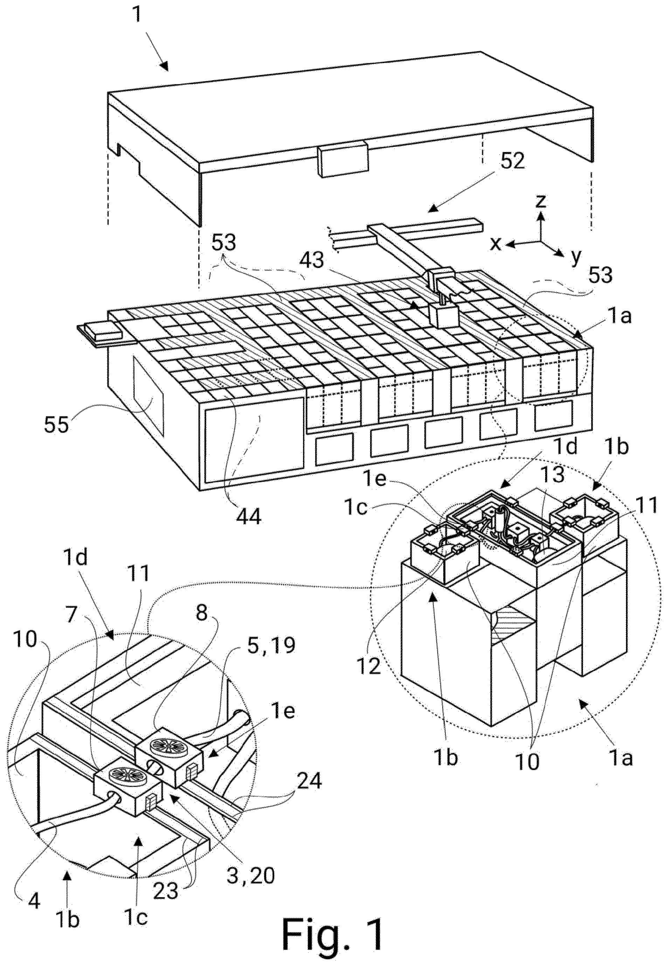

In Fig. 1, a preferred embodiment of a proposed integrated bioprocessing system 1 is shown exemplarily. The provided method may be performed with the proposed integrated bioprocessing system 1, which is respectively adapted.

The term "integrated bioprocessing system" refers to a system that enables the performance of several bioprocesses, whereby the bioprocesses are preferably performed at least partly simultaneously. The integrated bioprocessing system 1 may provide a central electronic process control to control the execution of all bioprocesses. The integrated bioprocessing system 1 may further provide shared infrastructure elements, like transport elements, media supply, supply of consumables and/or dedicated unit operation stations 1a for performing at least one, several or all unit operations of each bioprocess.

While performing the bioprocesses, in particular to perform the operation or operations on the cell culture, the integrated bioprocessing system 1 transfers a medium or several media, which is or are used in the bioprocess, through tubes 4, 5. The medium might in particular be the cell culture on which the respective bioprocess is going to be performed.

Therefore, within the integrated bioprocessing system 1, several tube connections 3 may be provided for transferring the medium, in particular cell culture, inside the integrated bioprocessing system 1. For example, the medium may be received and transported by receptacles 1b, which are movable from and to different unit operation stations 1a. At the unit operation stations 1a one or more unit operations might be performed. The unit operation itself may be performed by utilizing cartridges 1d, which are individually adjusted according to the unit operation to be performed. To transfer the respective medium from one of the receptacles 1b to one of the cartridges 1d or vice versa, these should be fluidically connected. Such a connection may be provided by connectable tubes 4, 5 of the receptacle 1b and the cartridge 1d. However, within the integrated bioprocessing system 1, there might be several other tube connections 3 for fluidically connecting components such that all kinds of media, in particular fluids, like liquids, for example liquid cell cultures, reagents, culture media, buffers, mixtures thereof or the like, can be transferred.

Currently, connections of components, particular connections via tubes, are often made by hand, which provides a risk of contamination and a risk of wrong connections due to mix-up of tubes, or are often preconfigured, which limits the flexibility of the known systems.

Proposed is, that the integrated bioprocessing system 1 comprises a tube connection system 2 for, particularly sterile, connecting tubes of the integrated bioprocessing system 1 as needed by welding. Thus, the welding takes place as demanded, for example, if an individual unit operation shall be performed. The tubes, which are going to be connected, may have a circular cross section.

The integrated bioprocessing system 1 comprises a first tube 4, a first tube holder 7 and a first fluidic structure 12 for receiving and/or providing the medium via the first tube 4. The first tube 4 is held by the first tube holder 7 in a defined position and is fluidically connected to the first fluidic structure 12, such that fluid, in particular the medium, can be guided to and from the first fluidic structure 12 by the first tube 4. The first fluidic structure 12 may contain the medium, in particular the cell culture, and/or may perform a unit operation on the medium, in particular the cell culture.

The term "fluidic structure" comprises all kinds of fluidic structure, which are able to receive and/or provide a medium, in particular cell cultures. Fluidic structures may be designed as a reservoir, wherein the medium is storable, or as an operational structure, in which a unit operation is performable.

The first tube 4, the first tube holder 7 and the first fluidic structure 12 are carried by a movable first carrier 10. The first carrier 10 may be designed as a container, wherein in particular the first fluidic structure is part of the container, or as a carrier, on which the first tube 4, the first tube holder 7 and the first fluidic structure 12 are arranged, in particular attached, as it is exemplarily depicted in fig. 1. The first tube 4 is in particular carried as a whole by the first carrier 10. The first carrier 10 may enable that the first tube 4 is transported to the welding location independently from a tube cutting unit 9 and/or a tube connection head 43.

Particularly because of the first carrier 10, the transport within the integrated bioprocessing system 1 is simplified. The first carrier 10 and with the first carrier 10 the first tube 4, the first tube holder 7 and the first fluidic structure 12 are transported to a welding location within the integrated bioprocessing system 1. At the welding location the welding takes place. The integrated bioprocessing systems 1 may comprise several welding locations, at which welding by the tube connection system 2 is possible. The transport of the first carrier 10 together with the first tube 4, the first tube holder 7 as well as the first fluidic structure 12 may be performed by the integrated bioprocessing system 1 automatically, in particular by a robotic mechanism 52 or preferably by a conveyor mechanism 53.

At the welding location, in a welding routine the tube connection system 2 connects the first tube 4 and a second tube 5 such that a, particularly sterile, tube connection 3 is formed by welding. The second tube 5 may be part of an apparatus for performing a unit operation, which comprises a second fluidic structure 13 and optionally a second tube holder 8. Alternatively, the second tube 5 may be carried by a second carrier 11. The second carrier 11 may carry, beside the second tube 5, a second tube holder 8 and a second fluidic structure 13, as exemplarily depicted in fig. 1.

After welding, the integrated bioprocessing system 1 transfers the medium from or to the first fluidic structure 12 of the first carrier 10 through the tube connection 3. In this way, previously not connected tubes 4, 5, and in particular fluidic structures 12, 13, may be, in particular automatically, connected while keeping them sterile.

The tube connection 3 is used to perform the bioprocess. Preferably, several tube connections 3 are used to perform a respective bioprocess. More preferably, different unit operations of a respective bioprocess are performed and tube connections 3 are formed and/or disconnected, as will be described below, between the unit operations. Exemplarily, a unit operation may be performed, then a tube connection 3 may be formed, in particular to transfer the cell culture to a location for the next unit operation, then the medium, in particular the cell culture may be transferred through the tube connection 3, the tube connection 3 may be disconnected and then another unit operation may be performed, for example, by a fluidic structure 13 connected to one of the tubes 4, 5, in particular on the cell culture. It is possible that between two unit operations, the tubes 4, 5 are transported within the integrated bioprocessing system 1, in particular by moving the respective carrier 10, 11.

As shown exemplarily in Fig. 1 and preferably, the first tube 4 and the second tube 5 are connectable such that the tube connection 3 is formed. The tube connection 3 fluidically connects components of the integrated bioprocessing system 1, like the first fluidic structure 12 and the second fluidic structure 13. Via the tube connection 3 media, in particular fluids, like liquids, preferably cell cultures, are transferable.

In the exemplarily shown and preferred embodiment, the first carrier 10, the first tube 4, the first tube holder 7 and the first fluidic structure 12 are part of a receptacle 1b. In the receptacle 1b, in particular the first fluidic structure 12, the medium as well as other fluids are transportable within the integrated bioprocessing system 1. Here and preferred, the second carrier 11, the second tube 5, the second tube holder 8 and the second fluidic structure 13 are part of a cartridge 1d. In the cartridge 1d, in particular the second fluidic structure 13, a unit operation is performable on the medium, in particular the cell culture. Here and preferably, the first tube 4 and the second tube 5 are designed as flexible tubes. Further preferably, the first tube 4 and the second tube 5 are equally designed, in particular regarding the tube diameter, the tube wall thickness and/or the tube material and/or the circular cross section.

The term "tube" in context with this application however has to be understood broadly such that "tube" comprises flexible tubes as well as rigid tubes like pipes, but also differently designed tube-elements, with which fluids can be transferred, like for example tube connectors or the like. Generally, a tube may be any media-conducting element, preferably an elongated, more preferably cylindrical, element. Preferably, the tubes are made of a plastic material, preferably of thermoplastic material, more preferably of thermoplastic elastomers.

It shall be generally noted that in context with this application the term "second" in combination with a respective component of the integrated bioprocessing system 1, like "second tube 5" or the like, or the tube connection system 2 have not necessarily to be understood that the embodiment does comprise at least two of the respective components. Moreover, the term "second" is chosen for distinction reason, like for distinguishing the first tube 4 from the second tube 5. For that reason, the integrated bioprocessing system 1 may comprise a second component, but does not comprise a respective first component. The same applies for the term "third".

Advantageously, the welding routine is performed, at least in parts automatically by the tube connection system 2. The automatically performed welding routine or automatically performed phases of the welding routine does or does not require manual input, actions or interactions from a user. Thus, the risk of contamination might be reduced and the overall bioprocess may be further automated. It is preferred that the welding routine is performed completely automatically.

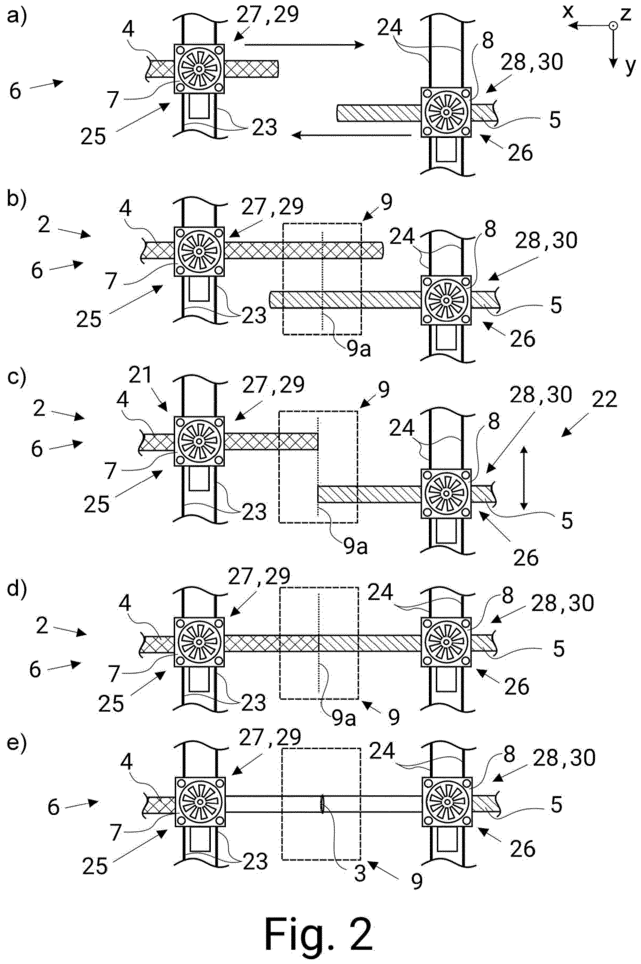

The welding routine may comprise different phases, namely an arrangement phase, a trimming phase and/or a welding phase. The welding routine is exemplarily shown in detail in Fig. 2a) to 2e). The different phases may be performed simultaneously, preferably at least partly simultaneously and/or sequentially. It is particularly preferred that the trimming phase is directly followed by the welding phase.

In a preferred embodiment, the welding routine comprises the arrangement phase. During the arrangement phase, the first tube 4 and the second tube 5 are being arranged relatively to each other at the welding location. Generally, relative arrangement of the first tube 4 and the second tube 5 may happen by direct or indirect movement of at least one of the tubes 4, 5. Direct movement might be performed by extrusion or retraction of the respective tube. Indirect movement might be performed by movement of a respective tube holder 7, 8 or by movement of the first carrier 10 and/or the second carrier 11 with the respective tube 4, 5. Here and preferably, the first tube 4 and/or the second tube 5 is or are moved, in particular only, in the axial direction of the first tube 4 and/or the second tube 5.

It is preferred, that during the arrangement phase the first tube 4 is, in particular automatically, moved, preferably extruded or retracted, in its axial direction and/or the second tube 5 is, in particular automatically, moved, preferably extruded or retracted, in its axial direction, as exemplarily shown in Fig. 2a). The axial direction of the first tube 4 and the axial direction of the second tube 5 are preferably inverse to each other, in particular if the first tube 4 is in a first cutting position and the second tube 5 is in a second cutting position. In Fig. 2a), the tubes 4, 5 are moved in the x-dimension, wherein the first tube 4 is moved in an inverse direction regarding to the moving direction of the second tube 5. Preferably and as shown in Fig. 2a), the first tube 4 is held by a first tube holder 7 and the second tube 5 is held by a second tube holder 8 in the arrangement phase.

Furthermore, it is possible, that the first tube 4 is moved in a transverse direction, in particular orthogonal direction, of its axis and/or that the second tube 5 is moved in a transverse, in particular orthogonal, direction of its axis. In Fig. 2a), the orthogonal direction of the axis of the first tube 4 and of the second tube 5 is within the y-dimension.

The arrangement phase may be at least partly performed by a tube arrangement unit 6, which in particular comprises the first tube holder 7 and/or the second tube holder 8. The movement of the first tube 4 and/or the second tube 5 may be performed directly, namely by directly moving the respective tube, for example by extruding and/or retracting the respective tube 4, 5 by a tube movement arrangement 27, 28, and/or indirectly, namely by moving the respective tube holder 7, 8, for example by a holder movement arrangement 21, 22, such that the respective tube 4, 5 is moved therewith in an axial direction and/or a transverse direction of the respective tube 4, 5. It is possible that the first tube 4 and/or the second tube 5 is moved by the respective tube holder 7, 8 in a transverse before the first tube 4 and/or the second tube 5 is moved in an axial direction by the tube movement arrangement 27, 28. Thus, the respective tube 4, 5 might be coarsely positioned.

During the arrangement phase, the first tube 4 and/or the second tube 5 may be moved into respectively the first cutting position and/or the second cutting position. In Fig. 2b), the first tube 4 and the second tube 5 are in their respective cutting positions. It is preferred, that the first tube 4 and the second tube 5 are arranged next to each other, in particular in parallel, if the first tube 4 is in the first cutting position and the second tube 5 is in the second cutting position. Preferably, the first tube 4 and the second tube 5 are at least partly overlapping each other if the tubes 4, 5 are in the respective cutting position. The first tube 4 and the second tube 5 may be arranged alongside each other in the respective cutting position.

It is preferred that reaching the respective cutting position is verified by a sensor. Exemplarily the tube connection system 2 may comprise one or more sensors that senses or sense the positions of the tubes 4, 5, in particular, if the tubes 4, 5 are in their respective cutting position. It is possible that the sensor or the sensors indicate if extrusion or retraction of the tubes 4, 5 has successfully started. Suitable sensors, for example photoelectric sensors, are well known in the art.

Furthermore, it is preferred that the method comprises temporarily closing the first tube 4 and/or the second tube 5 before the welding routine is performed. The first tube 4 and/or the second tube 5 might be temporarily closed by one or more closing elements 60, such as a clamp or the like. It is preferred that one tube closing element 60 is installed at either tube 4, 5. Preferably, the tube closing elements 60 are installed such that the tube connection 3 is formed between the tube closing elements 60. In this context, it is preferred that each tube closing element 60 is installed between the tube connection 3 and the respective tube holder 7, 8.

It is possible that the tube closing element 60 for closing the first tube 4 and/or the second tube 5 is or are provided by the first tube holder 7 and/or the second tube holder 8, respectively. Preferably, the first tube holder 7 and/or the second tube holder 8 might respectively comprise the closing element 60. Alternatively, the one or more closing elements 60 may be designed separately, for example as separate clamps or the like, and may be installed during the welding routine, for example by a robotic mechanism 52, preferably after the tubes 4, 5 are in their respective cutting positions.

It is preferred that after the welding routine is performed, an integrity testing routine for verifying the integrity of the tube connection 3 is performed. This verification check makes it possible to determine if the tube connection 3, which was formed by welding, is intact or unsafe. Intact means that the interior of the tubes remains separated from the outer atmosphere, whereby the sterility of the liquid within the tubes is maintained. Unsafe means that the tubes are not connected properly, particularly that the interior of the tubes is not completely sealed towards the outer atmosphere. If the seal is not complete, potential contaminants might enter the tubes from the outer atmosphere, which would compromise the sterility of the liquids to be transferred to the tubes. It is preferred that the temporary closing is reversed after the welding routine, preferably after an integrity testing routine was performed and the integrity of the tube connection 3 was detected. The temporary closing maintains sterility in the closed section of the tube 4, 5 if the welding fails. In this case, it is possible to perform a disconnection routine, as described in more detail in the following. Here and preferably, a sealing phase of the disconnection routine is performed before removing the closing elements 60. In this context, it is preferred to seal the first tube 4 and/or the second tube 5 between the first tube holder 7 and the respective closing element 60 and/or rather the second tube holder 8 and the respective closing element 60. In such a way, the sterility of the tubes 4, 5 can be ensured.

The integrity testing routine may be performed by a weld verification arrangement 49, which will be explained in detail below.

It is preferred that depending on the result of the integrity testing routine, if the integrity of the tube connection is verified, reversing the temporary closing of the first tube and/or the second tube is performed. Here, the medium can then be transferred from the first tube to the second tube or vice versa via the tube connection 3. However, if the integrity of the tube connection 3 is not verified, initiating an emergency routine for preserving the cell cultures is performed. In the emergency routine, the temporary closing is not reversed such that a contamination of the cell cultures via the unsafe tube connection 3 can be prevented. Thus, the sterility of the tubes and accordingly the sterility of the liquids within the tubes and the sterility of the liquids the tubes connect to can be secured. The emergency routine might comprise reporting a notification to a user and/or initiating a disconnection routine and/or initiating a disconnection routine that is followed by another welding routine. It is in particular possible, that the part of the first tube 4 and/or the second tube 5, which is or are respectively potentially exposed to the outside environment, remains closed by the closing element 60 or the closing elements 60, for example by clamping. Furthermore, it is possible, that the part of the first tube 4 and/or the second tube 5, which is or are respectively potentially exposed to the outside environment, is or are sealed and particularly cut off, for example by performing a disconnection routine. After sealing the tubes 4, 5 in the disconnection routine, which will be described in detail below, the closing element 60 or the closing elements 60 can be removed. After the disconnection routine was performed, another welding routine may be performed.

Alternatively or additionally, the welding routine comprises the trimming phase. During the trimming phase the first tube 4 and the second tube 5 are being cut at the welding location. It is preferred that the trimming phase follows the arrangement phase such that the tubes 4, 5 are being cut in their cutting positions. Preferably, the tubes 4, 5 are being cut by using a high temperature cutting procedure. The trimming phase may be at least partly performed by a tube cutting unit 9, which in particular comprises a blade 9a, a blade mount 9b and/or a blade heater 9c.

It is preferred that the tube connection system 2 comprises the tube cutting unit 9, wherein the tube cutting unit 9 comprises a blade 9a and preferably a blade heater 9c.

In context with the trimming phase, it is preferred that the trimming phase comprises a heating step, wherein during the heating step the blade heater 9c heats the blade 9a to a cutting temperature. This high temperature cutting procedure, wherein high temperatures are meant to be above 150°C, preferably above 250°C, further preferably above 350°C, provides decontamination during the welding routine. Furthermore, cutting of the tubes 4, 5 might be simplified, because the tubes 4, 5, which are preferably plastic tubes, are melted during cutting by the heated blade 9a.

Here and preferably the trimming phase comprises an alignment step, wherein the tube cutting unit 9 is aligned relatively to the first tube 4 and/or the second tube 5. The alignment of the tube cutting unit 9 relatively to the first tube 4 and/or the second tube 5 can be made such that the tube cutting unit 9 is moved and/or such that the first tube 4 and/or the second tube 5 is or are moved. Preferably, the first tube 4 is in the first cutting position and/or the second tube 5 is in the second cutting position, when the alignment step takes place. Here, the tube cutting unit 9 may be moved for alignment. It is preferred that the alignment step follows the heating step or that the heating step follows the alignment step or that the alignment step is performed at least partially simultaneously to the heating step and vice versa.

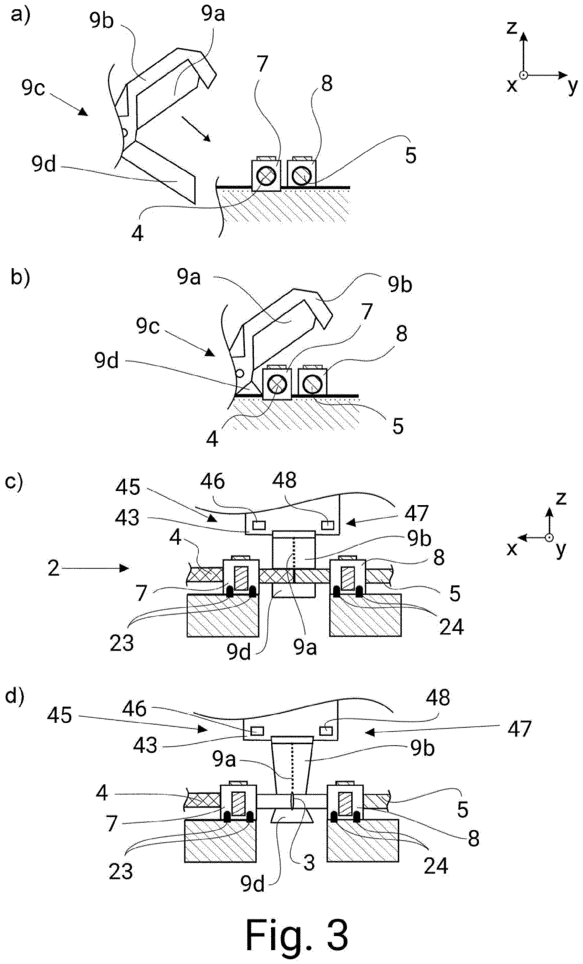

In Fig. 2b) the heating step as well as the alignment step of the trimming phase are exemplarily shown in a preferred embodiment. The first tube 4 and the second tube 5 are in the respective first cutting position or second cutting position. The tubes 4, 5 are being held by the tube holders 7, 8. The tube cutting unit 9 is aligned relatively to the tubes 4, 5, wherein the tube cutting unit 9 itself is moved during the alignment step, see Fig. 2b) compared to Fig. 2a). The tube cutting unit 9 is aligned such that the blade 9a is transversely, in particular orthogonally, orientated to the first tube 4 and/or the second tube 5. The blade 9a is heated by the blade heater 9c of the tube cutting unit 9 during the heating step. Another example of the alignment step is shown in Fig. 3a), wherein the tube cutting unit 9 is moved relative to the tubes 4, 5, as exemplarily indicated by the arrow in Fig. 3a). Fig. 3b) shows exemplarily the heating step, wherein the blade 9a is heated by the blade heater 9c.

Further preferably in context with the trimming phase, the trimming phase comprises a cutting step, wherein the first tube 4, in particular being in the first cutting position, and/or the second tube 5, in particular being in the second cutting position, is or are being cut by the tube cutting unit 9, in particular by the blade 9a.

During the cutting step the tubes 4, 5 are being cut. Preferably, the first tube 4 and the second tube 5 are being cut at least partly, preferably completely, simultaneously. It is preferred that the cutting step follows the heating step and/or alignment step. Further preferably, the blade 9a is heated to a certain temperature by the blade heater 9c during the cutting step. The tubes 4, 5 may be at least partially melted during the cutting step. It is possible that during the trimming phase the first tube 4 and/or the second tube 5 is or are squeezed in a squeeze direction and the cutting is performed in transverse direction, in particular orthogonally, to the squeeze direction. In this way, the cutting might be optimized and simplified. Due to squeezing of the tubes 4, 5, the cross section of the respective tube 4, 5 comprises temporarily a short and a long side, whereat cutting may be simplified by cutting along the long side.

In Fig. 2c) the cutting step of the trimming phase is exemplarily shown in the preferred embodiment. The heated blade 9a cuts the first tube 4 and the second tube 5 at least partly simultaneously. During the cutting step, the first tube 4 and the second tube 5 are generally opened one-sided, but the blade 9a blocks the openings of the tubes 4, 5 towards the surrounding atmosphere. As a result, contamination can be prevented, in particular because of the temperature of the blade 9a. Another example of the cutting step is exemplarily shown in Fig. 3c), wherein the blade 9a cuts through the tubes 4, 5. As it can be seen and preferably, the tube cutting unit 9 comprises a cutting table 9d, which faces the blade 9a. The cutting table 9d may support the cutting process by the blade 9a.

Additionally or alternatively to the trimming phase, the welding routine comprises the welding phase. During the welding phase the first tube 4 and the second tube 5 are being brought in contact and merged together at the welding location such that the tube connection 3 is formed. The welding phase preferably follows the arrangement phase and/or the trimming phase. The welding phase, in particular, directly follows the trimming phase such that after cutting the first tube 4 and the second tube 5 within the trimming phase, the first tube 4 and the second tube 5 are contacted and merged together within the welding phase. The welding phase is in particular partly performed by the tube arrangement unit 6 and the tube cutting unit 9. Contacting and merging of the tubes 4, 5 is preferably performed by the first tube holder 7 and/or the second tube holder 8, which, for example, extrude the first tube 4 and/or the second tube 5 such that these are contacted. The welding phase is exemplarily shown in Fig. 2c), 2d) and 2e).

In context with the welding phase and advantageously, the welding phase comprises a contacting step. Preferably, during the contacting step the first tube 4 and the second tube 5 are axially aligned. Axial alignment of the tubes 4, 5 may take place along the tube cutting unit 9, in particular along the blade 9a. Here, the openings of the tubes 4, 5 being cut may be covered by the blade 9a. Preferably, the first tube 4 and the second tube 5 are axially aligned by movement of the first tube holder 7 and/or the second tube holder 8 respectively, in particular by the first holder movement arrangement 21 and/or the second holder movement arrangement 22. The first tube holder 7 and/or the second tube holder 8 may be movable independently from the tube connection head 43 and the tube cutting unit 9.

Alternatively or additionally, during the contacting step the tube cutting unit 9 is at least partly moved, in particular transversely, preferably orthogonally, to the tubes 4, 5, such that the first tube 4 and the second tube 5 can be brought in contact. Preferably the blade 9a is moved, in particular transversely, preferably orthogonally, to the tubes 4, 5, such that the first tube 4 and the second tube 5 can be brought in contact. It is preferred that this movement of the tube cutting unit 9 is performed after the axial alignment of the tubes 4, 5. Furthermore, alternatively or additionally, during the contacting step the first tube 4 and the second tube 5 are brought in contact, in particular by axially moving the first tube 4 and/or the second tube 5, preferably by the first tube movement arrangement 27 and/or the second tube movement arrangement 28. This may be performed after axial alignment of the tubes 4, 5 and/or after movement of the tube cutting unit 9. Preferably, axially moving of the first tube 4 is made by moving the first tube holder 7 and/or moving of the second tube 5 is made by moving the second tube holder 8. Alternatively and likewise preferred, axially moving of the first tube 4 is made by the first tube holder 7 such that the first tube 4 is extruded and/or retracted and/or of the second tube 5 is made by the second tube holder 8 such that the second tube 5 is extruded and/or retracted. During the contacting step the first tube 4 and the second tube 5 are brought in contact and merged together, in particular since the tubes 4, 5 are heated up by the heated blade 9a during the trimming phase. During the contacting step the, in particular sterile, tube connection 3 is formed.

In Fig. 2c), 2d) and 2e) the welding phase is exemplarily depicted. Here and preferably, the first tube 4 and the second tube 5 are axially aligned by, in particular horizontal, movement of the first tube holder 7 and/or the second tube holder 8, see Fig. 2c) compared to Fig. 2d). The movement of the first tube holder 7 and/or the second tube holder 8 may be performed transversely, in particular orthogonal, to the axis of the first tube 4 and/or the second tube 5. During the axially alignment, the blade 9a is kept in place such that the first tube 4 and/or the second tube 5 are moved along the blade 9a, in particular contacting the blade 9a during this movement. In particular after the axial alignment, the blade 9a is moved, in particular transversely, in particular orthogonally, to the tubes 4, 5, such that the tubes 4, 5 can be contacted. In Fig. 2d), the blade is moved upward in a vertical direction. In Fig. 2e), the first tube 4 and the second tube 5 are brought in contact, by axial movement of at least one of the tubes 4, 5, and are merged together. Thus, the tube connection 3 is formed. The welding phase is furthermore exemplarily depicted in Fig. 3c) and 3d).

It is possible that the tube connection 3 is opened after welding is performed. The tube connection 3 may be opened by an external force, which is applied at the tube connection 3 on the outer surface of the first tube 4 and the second tube 5. The external force may be applied by the tube cutting unit 9 or the tube welding head 43 or another robot or the like. The external force may be applied perpendicular to the axis of the tubes 4, 5. Alternatively, the tube connection 3 may be opened by applying inner pressure to the tubes 4, 5. This might be realized by a pumping arrangement 54.

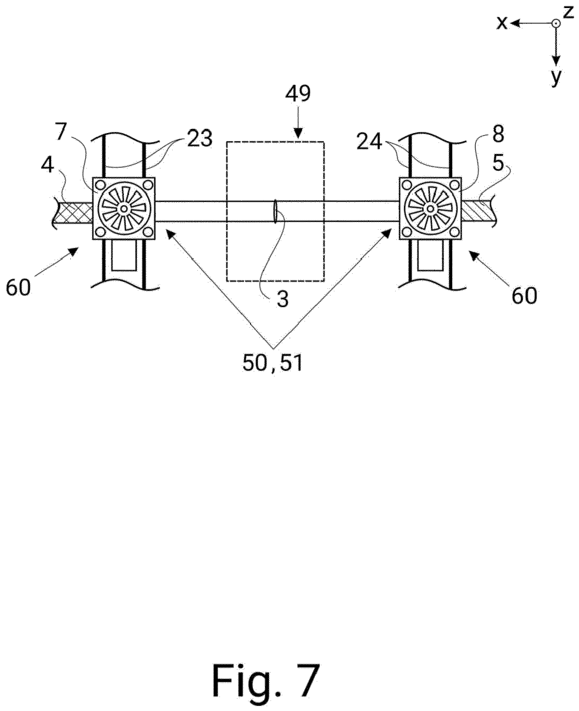

It is furthermore preferred that the method comprises temporarily closing the first tube 4 and/or the second tube 5 before the welding routine or before the welding phase is performed. The first tube 4 and/or the second tube 5 might be closed by a clamp, in particular a clamp which is independently designed from a tube connection head 43, or the like. It is preferred that after the welding routine or after the welding phase is performed, an integrity testing routine for verifying the integrity of the tube connection is performed, as exemplarily shown in Fig. 7. This verification check makes it possible to determine if the tube connection 3, which was formed by welding, is intact or unsafe. Depending on the result of the integrity testing routine, if the integrity of the tube connection is verified, reversing the temporary closing of the first tube 4 and/or the second tube 5 is performed. Then, the medium can be transferred through the tube connection 3. However, if the integrity of the tube connection 3 is not verified, initiating an emergency routine for preserving the cell cultures is performed. Here, the temporary closing is not reversed such that a contamination of the cell cultures via the unsafe tube connection 3 can be prevented. The emergency routine might comprise reporting a notification to a user and/or initiating a disconnection routine and/or initiating another welding routine.

It is furthermore preferred that the tube connection system 2 comprises a tube disconnection arrangement 14. The tube disconnection arrangement 14 preferably comprises a sealing unit 15 and/or a disconnecting unit 17.

In a further preferred embodiment, the method comprises a disconnection routine, wherein the first tube 4 and the second tube 5 are disconnected. By the disconnection routine the tubes 4, 5 may be fluidically and/or physically disconnected. "Fluidically disconnected" means that fluid cannot be transferred through the tubes 4, 5, because, for example the tubes 4, 5 are being sealed. "Physically disconnected" means that the tubes 4, 5 are fully disconnected and might be handled, for example moved, independently from each other. The disconnection routine might be performed by the tube disconnection arrangement 14 of the tube connection system 2.

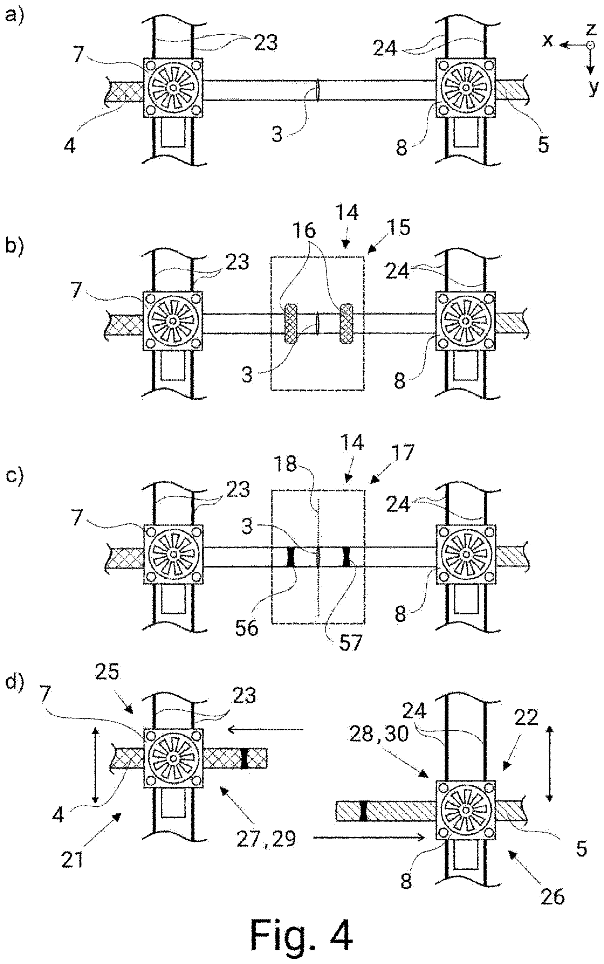

It is preferred that the disconnection routine comprises a sealing phase, wherein the first tube 4 and/or the second tube 5, which are in particular currently connected, is or are sealed such that a first sealing 56 and/or a second sealing 57 are formed. This is exemplarily depicted in Fig. 4. It is preferred that during the sealing phase, the first tube 4 and/or the second tube 5 are sealed by compression and heat. Here, the first tube 4 and/or the second tube 5 is compressed, in particular transversely to the respective tube axis, and heated such that the first tube 4 and/or the second tube 5 is partly melted and sealed, in particular self-sealed. The sealing phase might be performed by the tube disconnection arrangement 14, in particular by a sealing unit 15 of the tube disconnection arrangement 14. The sealing unit 15 may comprise a sealing surface 16 for compressing and heating. The sealing unit 15 may be designed independently from the tube cutting unit 9. The sealing surface 16 may be designed independently from a disconnecting blade 18.

Advantageously, the disconnection routine comprises a disconnecting phase, wherein the first tube 4 and/or the second tube 5 are cut, in particular between the first sealing 56 and the second sealing 57. It is also possible, to cut the first tube 4 or the second tube 5 in the range of the respective sealing 56, 57 itself. Thus, the tubes 4, 5 are being physically disconnected. The disconnecting phase may be performed by the tube disconnection arrangement 14, in particular by a disconnecting unit 17 of the tube disconnection arrangement 14.

Fig. 4a), 4b), 4c) and 4d) show exemplarily the disconnection routine. Here and preferably, the first tube 4 and the second tube 5 are physically and fluidically connected first, as depicted exemplarily in Fig. 4a). Preferably and, for example, shown in Fig. 4b), during the disconnection routine the first tube 4 and the second tube 5 are sealed at least partly simultaneously by the sealing unit 15 of the tube disconnection arrangement. However, the sealings might be performed sequentially, namely one after another, too. The sealing unit 15 comprises two sealing surfaces 16, which compress the tubes 4, 5 and are heated such that the tubes 4, 5 are self-sealed, as exemplarily shown in Fig. 4c). As a result, the first sealing 56 and the second sealing 57 occur. After sealing of the tubes 4, 5 in the sealing phase, the tubes 4, 5 are being cut in the disconnecting phase by the disconnecting unit 17, in particular by a disconnecting blade 18, as also exemplarily shown in Fig. 4c). Preferably, the disconnecting blade 18 is heated to simplify the cutting. Here and preferably, the sealing phase is performed before the disconnecting phase. Alternatively, it is also possible that the sealing phase is performed after the disconnecting phase, for example if the disconnecting blade 18 cuts the tubes 4, 5 and temporarily seals the resulting openings. Here and preferably, the disconnecting blade 18 cuts the first tube 4 and the second tube 5 in a cutting plane, which is perpendicular to the axis of the tubes 4, 5. The cutting plane may be transversely orientated to the sealing surfaces 16 and/or the first sealing 56 and/or the second sealing 57.

It is preferred that the method furthermore comprises connecting the first tube 4 or the second tube 5 and a third tube 19 of the integrated bioprocessing system 1 by the tube connection system 2 such that a further tube connection 20 is formed, wherein the further tube connection 20 is formed in a welding routine. The welding routine may be performed as described beforehand in context of the tube connection 3 of the first tube 4 and the second tube 5. In particular, the same tube connection system 2 is used for connecting the first tube 4 or the second tube 5 and the third tube 19. It is possible that the first tube 4 or the second tube 5 is welded in a welding routine multiple times to different tubes 4, 5, 19. This is an interesting advantage of using a welding system as tubes 4, 5, 19 can be cut and welded multiple times easily if enough tube length is present.

The third tube 19 may be carried by a third carrier. The third carrier may carry a third tube holder and a third fluidic structure. It is possible that the third carrier, the third tube 19, the third tube holder and the third fluidic structure are part of a receptacle 1b or a cartridge 1d.

The different tubes 4, 5, 19 may be from different cartridges 1d and/or different receptacles 1b. It is thus possible that a cartridge 1d is connectable to different receptacles 1b, in particular one after another. It is further possible that a receptacle 1b is connectable to different cartridges 1d, preferably one after another. Furthermore, it is also possible that a cartridge 1d comprises several tubes 4, 5, 19, which are connected to tubes 4, 19 of a receptacle 1b or to tubes 4, 5, 19 of several receptacles 1b. Here, the cartridge 1d may be connected to the receptacle 1b via several tube connections 3, 20 or may be connected to several receptacles 1b via several tube connections 3, 20.

For example, a cartridge 1d comprises a tube 4, 5, 19 which is connected to a tube 4, 5, 19 of a receptacle 1b such that a tube connection 3, 20 is formed. A medium or media may be transferred via the tube connection 3, 20 and a unit operation might be performed on the cartridge 1d. The tubes 4, 5, 19 may be disconnected thereafter, in particular by performing a disconnection routine. Afterwards, the cartridge 1d and/or the receptacle 1b may be transported to another location of the integrated bioprocessing system 1, in particular to another welding location. Then, another tube connection 3, 20 can be formed, in particular by performing a welding routine, by connecting the tube 4, 5, 19 of the cartridge to another tube 4, 5, 19 of the receptacle 1b or to another tube 4, 5, 19 of another receptacle 1b.

It is possible that first, a first receptacle 1b comprising the liquid immune cell culture is connected to the cartridge 1d using a first set of tube holders 7, 8 and connecting a tube 4, 19 of the first receptacle 1b to a tube 5, 19 of the cartridge 1d. The first receptacle 1b may comprise a first fluidic structure 12, which initially contains media, in particular the cell culture. The cartridge 1d, in particular a fluidic structure 13 of the cartridge, may receive the media from the first receptacle 1b. It may then be the case, that, for example, for performing counter-flow centrifugation in the fluidic structure 13 of the cartridge 1d, a tube 4, 19 of a second receptacle 1b with a further fluidic structure comprising a buffer is connected to the cartridge 1d using a second tube 5, 19 of the cartridge 1d that is situated in a different holder 8 of the cartridge 1d.

Alternatively, the tube 4, 19 of the second receptacle 1b may be connected to the first tube 5, 19, of the cartridge 1d, when the tube connection 3, 20 between the first receptacle 1b and the cartridge 1d was subsequently disconnected. After performing a unit operation, the liquid immune cell culture may be transferred from the second fluidic structure 13 of the cartridge 1d to a third receptacle 1b, in particular to a further fluidic structure of the third receptacle 1b, while waste material may be transferred from the cartridge 1d to a fourth receptacle 1b. Here it may also be the case that two or more holders 8 are arranged on the same rail. It is also possible that a receptacle 1b comprises several tubes 4, 19, which are connected one after another to a tube 5, 19 of a cartridge 1d. Thus, a receptacle 1b may comprise different media, for example contained in different vessels of the fluidic structure, which are transferable to the cartridge 1d through the different tubes 4, 19. Exemplarily, a receptacle 1b may comprise a first vessel comprising a liquid (e.g., buffer for cell washing) and a second vessel for receiving a liquid (e.g., waste from the cell washing step). In this case, each vessel may comprise a tube 4, 19 and each tube 4, 19 may be situated in a holder 7. Both holders may be arranged on the same rail of the receptacle 1b. In a first step, the buffer may be transferred from the first vessel to a centrifuge unit of the fluidic structure 13 located in the cartridge 1d by utilizing a first tube 5, 19 of the cartridge 1d. After or during centrifugation, the waste from the centrifugation may be transferred from the cartridge 1d by utilizing a second tube 5, 19 of the cartridge 1d. This second tube 5, 19 may be connected to the tube 4, 19 that belongs to the second vessel of the receptacle 1b.

Preferably, several tube connections 3, 20 are formed by several welding routines, wherein in particular within the integrated bioprocessing system 1 one, in particular the same, or more tube connection systems 2 are performing the welding routines. Welding routines are preferably performed before, during and/or after the performance of bioprocesses.

It is particularly preferred that during a bioprocess being performed on a cell culture, the welding routine is performed at least once, preferably at least once for each unit operation being performed on the cell culture. It is possible that during the welding routine a cartridge 1d and a receptacle 1b are being connected, in particular by the tube connection 3. Preferably, after performing the welding routine at least one unit operation of the bioprocess is performed, in particular on the cartridge 1d. The receptacle 1b may comprise a medium, in particular the liquid immune cell culture, which is used in the bioprocess, in particular in the unit operation, and thus is transferred to the cartridge 1d. After performing the at least one unit operation, a disconnection routine is performed such that particularly the cartridge 1d and the receptacles 1b are being disconnected. The cartridge 1d and/or the receptacles 1b may be moved within the integrated bioprocessing system 1 thereafter. The receptacle 1b, exemplarily comprising the liquid immune cell culture, may be moved to another unit operation station 1a, in particular to perform another unit operation on the liquid immune cell culture by utilizing a different cartridge 1d.

Generally, it is also possible, that a tube connection 3 is formed for extending the first tube 4. Here, the second tube 5 is designed as an extension tube, which is welded to the first tube 4 as described. It is also possible that a tube connection 3 is formed for extending the second tube 5. Here, the first tube 4 is designed as an extension tube, which is welded to the second tube 5 as described. However, in one embodiment, a tube 4, 5 may be extended during the disconnection routine. For this, before disconnecting, the location of the tube connection 3 is changed, in particular by a tube holder 7, 8, such that one of the tubes 4, 5 is extended with a section of the other tube 4, 5. The other tube 4, 5 is therefore shortened. The integrated bioprocessing system 1 may comprise a welding plan including steps for shortening and extending a tube 4, 5 with consecutive welds and disconnects such that a target length interval of the tube 4, 5 is kept.

As a second teaching, the integrated bioprocessing system 1, as exemplarily depicted in Fig. 1, is provided. As the integrated bioprocessing system 1 is adapted to perform the provided method, all explanations and features given before are fully applicable. Vice versa, all following explanations and features are fully applicable to the proposed method.

The integrated bioprocessing system 1 is adapted for, in particular automatically, performing bioprocesses on cell cultures, wherein while performing the bioprocesses a medium used in the bioprocess, in particular the cell cultures, is transferable by the integrated bioprocessing system 1 through tubes 4, 5. The bioprocessing system 1 comprises a first tube 4 for transferring medium, in particular fluid, and a second tube 5 for transferring medium, in particular fluid. The first tube 4 is held by a first tube holder 7 and is fluidically connected to a first fluidic structure 12 for receiving and/or providing the medium. It is preferred that the first tube holder 7 holds a single tube 4.

The integrated bioprocessing system 1 comprises a movable first carrier 10, wherein the first tube 4, the first tube holder 7 and the first fluidic structure are carried by the first carrier 10. The first carrier 10 and with the first carrier 10 the first tube 4, the first tube holder 7 and the first fluidic structure are transportable to a welding location within the integrated bioprocessing system 1. The first carrier acts thus as a transportation vehicle for the first tube 4 and the integrated bioprocessing system 1 comprises a tube connection system 2 for connecting the first tube 4 and the second tube 5 in a welding routine at the welding location such that a, in particular sterile, tube connection 3 is formed by welding, wherein the medium is transferable through the tube connection 3 from or to the first fluidic structure 12 of the first carrier 10 after welding. Here and preferably, the medium is transferred through the tubes 4, 5 and the tube connection 3 as part of the bioprocess or bioprocesses.

It is preferred that the integrated bioprocessing system 1 comprises a robotic mechanism 52. The robotic mechanism 52 might be part of the tube connection system 2. The robotic mechanism 52 might be involved in the welding routine, but however might be in particular involved in other actions in the integrated bioprocessing system 1.

It is preferred that the second tube 5 is held by a second tube holder 8 and is fluidically connected to a second fluidic structure 13 for receiving and/or providing the medium. It is preferred that the second tube holder 8 holds a single tube 5. After welding, medium is transferable through the tube connection 3 from or to the second fluidic structure 13 of the second carrier. When the first tube 4 and the second tube 5 are connected, medium can be transferred between the first fluidic structure 12 and the second fluidic structure 13.

The second tube 5 may be part of an apparatus for performing a unit operation. In this case, the second tube 5 may be held by a second tube holder 8, which is, for example, arranged on or attached to the apparatus. The apparatus may comprise a second fluidic structure 13. However, it is more preferred that the integrated bioprocessing system 1 comprises a movable second carrier 11, wherein the second tube 5, the second tube holder 8 and the second fluidic structure 13 are carried by the second carrier 11. The second carrier 11, the second fluidic structure 13, the second tube 5 and the second tube holder 8 may be part of a receptacle 1b or particularly a cartridge 1d. The second carrier 11 and with the second carrier 11 the second tube 5, the second tube holder 8 and the second fluidic structure 13 are transportable to the welding location. The second carrier 11 may be transportable independently from the first carrier 10 and/or the tube connection head 43 and/or the tube cutting unit 9

Furthermore it is possible that the integrated bioprocessing system 1 comprises a conveyor mechanism 53. The conveyor mechanism 53 may be part of the tube connection system 2. The conveyor mechanism 53 might be involved in the welding routine, but may also be involved in other actions in the integrated bioprocessing system 1. The conveyor mechanism 53 may transport the first carrier 10 and/or the second carrier 11 and/or the cartridge 1d or cartridges 1d and/or the receptacle 1b or receptacles 1b within the integrated bioprocessing system 1, in particular to the welding location.

Alternatively or additionally, the robotic mechanism 52 is designed to transport the cartridge 1d or cartridges 1d and/or the receptacle 1b or receptacles 1b within the integrated bioprocessing system 1. In particular, the robotic mechanism 52 may handle the cartridge 1d or cartridges 1d and/or the receptacle 1b or receptacles 1b by picking, moving and releasing. The robotic mechanism 52 may be designed as a rail robotic mechanism 52, which comprises different moving axis or axes for linear movement in one, two or three dimensions (cartesian robotic mechanism). The robotic mechanism 52 might be designed as a robotic arm as an alternative.

The tube connection system 2 of the integrated bioprocessing system 1 preferably comprises a tube arrangement unit 6 for arranging, in an arrangement phase of the welding routine, the first tube 4 and the second tube 5 relatively to each other in at least an axial direction of the first tube 4 and/or the second tube 5, such that the first tube 4 and the second tube 5 can be cut. Preferably, the first tube 4 and the second tube 5 are arranged in the arrangement phase unidimensional or bidimensional. It is preferred, that the first tube 4 and/or the second tube 5 is or are moved in at least an axial direction of the first tube 4 and/or the second tube 5. It is preferred that this arrangement is performed automatically. The tube connection system 2 furthermore comprises a tube cutting unit 9 for cutting, in a trimming phase of the welding routine, the first tube 4 and the second tube 5. Preferably, the tube arrangement unit 6 is designed such that, in a welding phase of the welding routine, the first tube 4 and the second tube 5 are brought in contact and merged together such that the tube connection 3 is formed.

In context with this application, "dimension" is related to a three-dimensional cartesian coordinate system, like the x-, y- or z-dimension. "Unidimensional", "bidimensional" and "tridimensional" means in context with movement that a movement in one, two or three dimensions can be taken.

The tube connection system 2 connects the first tube 4 and the second tube 5 of the integrated bioprocessing system 1 in the welding routine, wherein a tube connection 3 is formed by welding. The tube connections system 2 comprise the tube arrangement unit 6, which arranges in an arrangement phase of the welding routine, the first tube 4 and the second tube 5 relatively to each other in at least an axial direction of the first tube 4 and/or the second tube 5 such that the first tube 4 and the second tube 5 can be cut. Furthermore, the tube connection system 2 comprises the tube cutting unit 9, which cuts the first tube 4 and the second tube 5 in the trimming phase of the welding routine. The tube arrangement unit 6 in the welding phase of the welding routine brings the first tube 4 and the second tube 5 in contact and merges them together such that the tube connection 3 is formed. It is preferred that the tube arrangement unit 6 is designed separately from the tube cutting unit 9. Thus, arrangement of the tubes 4, 5 may be performed independently from the tube cutting unit 9.

It is preferred that the tube arrangement unit 6 and the tube cutting unit 9 are designed such that the welding routine is performed, at least in parts, preferably completely, automatically. Thus, the risk of contamination might be reduced and the tube connection process may be further automated. Furthermore, the automatically performable welding routine may provide advantages, if the provided tube connection system 2 is applied in the provided integrated bioprocessing system 1, since the automation of the integrated bioprocessing system 1 can be improved. In particular, the first carrier 10 and/or the second carrier 11 enable automatic transportation of the first tube 4 and/or the second tube 5 within the integrated bioprocessing system 1.

In a preferred embodiment, the tube arrangement unit 6 comprises the first tube holder 7 for holding the first tube 4 and the second tube holder 8 for holding the second tube 5. The first tube holder 7 and/or the second tube holder 8 is or are part of the tube arrangement unit 6. As it is shown exemplarily in Fig. 2a) to 2e) and preferably, the first tube holder 7 holds the first tube 4 at least during the overall welding routine. The same applies for the second tube holder 8, which holds the second tube 5 at least during the overall welding routine. In general, and preferred, the first tube holder 7 and the second tube holder 8 are designed such that the first tube 4 and the second tube 5 can be presented for performing the welding routine on the tubes 4, 5 to form the tube connection 3. Since the first tube holder 7 is preferably arranged on or attached to the first carrier 10 and/or the second tube holder 8 is preferably arranged on or attached to the second carrier 11, the tube holders 7, 8 hold the tubes 4, 5 in a respective defined position, in particular before and after the welding routine is performed.

It is preferred that the tube arrangement unit 6 furthermore comprises a first holder movement arrangement 21 for moving the first tube holder 7, preferably, unidimensional, bidimensional or tridimensional, further preferably transverse, preferably orthogonal, to the axial direction of the first tube. The first holder movement arrangement 21 may enable to, in particular horizontally, move the first tube holder 7, wherein the first tube 4 may be moved with the first tube holder 7. Additionally or alternatively, the tube arrangement unit 6 comprises a second holder movement arrangement 22 for moving the second tube holder 8, preferably, unidimensional, bidimensional or tridimensional, further preferably transverse, preferably orthogonal, to the axial direction of the second tube 5. The second holder movement arrangement 22 enables to, in particular horizontally, move the second tube holder 8, wherein the second tube 5 may be moved with the second tube holder 8, as it is exemplarily depicted in Fig. 2c).

Regarding the first holder movement arrangement 21 and/or the second holder movement arrangement 22, it is preferred that the first holder movement arrangement 21 comprises a first holder guide 23 and/or that the second holder movement arrangement 22 comprises a second holder guide 24. The holder guides 23, 24 enable a predefined guided movement of the respective tube holder 7, 8. The first holder guide 23 and/or the second holder guide 24 may be designed respectively as a rail unit. As it can exemplarily be seen in Fig. 1 and 2a) to 2e) and preferably, at least one of the tube holders 7, 8 is movably mounted on a holder guide 23, 24, which are designed respectively as a rail unit, such that at least one of the tube holders 7, 8 can be moved horizontally unidimensionally, see for example Fig. 2c). In the example according to Fig. 2c), the second tube holder 8 can be moved in the y-direction of the Cartesian coordinate system, transversely to the second tube 5. The holder guides 23, 24, which are designed as rail units, may comprise respectively one or two rails. It is possible that the first tube holder 7 and/or the second tube holder 8 are attached via the first holder guide 23 to the first carrier 10 or rather the second holder guide 24 to the second carrier 11. Thus, the holders guides 23, 24 may fulfill a double function.

Preferably, the first holder movement arrangement 21 comprises a first holder drive unit 25 and/or the second holder movement arrangement 22 comprises a second holder drive unit 26. The holder drive unit 25, 26 drives the respective movement of the tube holder 7, 8, in particular on the holder guides 23, 24. It is possible, that the first holder drive unit 25 is at least partly, in particular fully, arranged on the first tube holder 7 and/or the second holder drive unit 26 is at least partly, in particular fully, arranged on the second tube holder 8. Thus, the tube holders 7, 8 might be self-driven. Alternatively, in another preferred embodiment, the first holder drive unit 25 and/or the second holder drive unit 26 is or are designed separately from the first tube holder 7 and/or the second tube holder 8. It is possible, that the first holder drive unit 25 and/or the second holder drive unit 26 is or are temporarily connectable to the respective tube holder 7, 8, such that the first tube holder 7 and/or the second tube holder 8 can be driven, in particular such that the respective tube holder 7, 8 may be moved on the holder guide 23, 24. The first holder drive unit 25 and/or the second holder drive unit 26 may be brought, in particular by the robotic mechanism 52, to the first tube holder 7 and/or the second tube holder 8 as needed. In another embodiment it is possible that the first holder drive unit 25 and/or the second holder drive unit 26 may be part of the robotic mechanism 52 such that the first tube holder 7 and/or the second tube holder 8 is or are moved by the robotic mechanism 52.

It is particularly preferred that the first holder drive unit 25 and/or the second holder drive unit 26 can be used to drive several different first tube holders 7 and/or several different second tube holders 8 within the integrated bioprocessing system 1. The different first tube holders 7 and/or the different second tube holders 8 may be part of different cartridges 1d and/or different receptacles 1b.

Generally, it is possible that a single second carrier 11 carries several second tube holders 8. It is also possible that a single cartridge 1d comprises several second tube holders 8. Each second tube holder 8 may hold a second tube 5, such that the second carrier 11 carries or rather the cartridge 1d comprises several second tubes 5. Generally, it is also possible that a single first carrier 10 carries several first tube holders 7, wherein each first tube holder 7 holds a first tube 4. It is possible that a single receptacle 1b comprises several first tube holders 7, wherein each first tube holder 7 holds a first tube 4. Thus, the several first tubes 4 and/or the several second tubes 5 might be connected one after another to transfer a medium, like the liquid immune cell culture, a washing buffer, waste material or the like.

Generally, multiple tube connections 3 may be formed as needed. The multiple tube connections 3 may be formed simultaneously, in parts simultaneously or one after another. The tube connections 3 may be formed between different first tubes 4 and different second tubes 5, which are in particular held by different first tube holders 7 and different second tube holders 8. The different first tubes 4 might be carried on a single first carrier 10. In particular the different first tubes 4 may each have different functionalities, for example, media ingress and media egress or the like. The different second tubes 5 might be carried on a single second carrier 11. In particular the different second tubes 5 may each have different functionalities, for example, media ingress and media egress or the like.

Beside the movement design of the tube holders 7, 8, it has turned out to be advantageous if the tube arrangement unit 6, in particular the first tube holder 7, comprises a first tube movement arrangement 27 for moving the first tube 4 held by the first tube holder 7 at least unidimensionally, in particular in its axial direction. Preferably the first tube 4 held by the first tube holder 7 can be moved relatively to the first tube holder 7 and the first carrier 10. Alternatively or additionally, the tube arrangement unit 6, in particular the second tube holder 8, comprises a second tube movement arrangement 28 for moving the second tube 5 held by the second tube holder 8 unidimensionally, preferably in its axial direction, wherein preferably the second tube 5 held by the second tube holder 8 is relatively moved to the second tube holder 8 and the second carrier 11. It is preferred, that the first tube 4 can be extruded and/or retracted by the first tube movement arrangement 27 and/or that the second tube 5 can be extruded and/or retracted by the second tube movement arrangement 28. For example, as it is depicted in Fig. 2a) and 4d) and preferably, the first tube 4 is horizontally extruded or retracted by the first tube holder 7. In the embodiment, shown in the Fig. 2a) and 4d), the first tube 4 is extruded or retracted unidimensionally in the x-direction of the Cartesian coordinate system. The second tube 5 is horizontally extruded or retracted by the second tube holder 8. In the embodiment, shown in the Fig. 2a), 4d), the second tube 5 is extruded or retracted unidimensionally in the x-direction of the Cartesian coordinate system. For driving this movement of the respective tube 4, 5, it is preferred that the first tube movement arrangement 27 comprises a first tube drive unit 29 and/or the second tube movement arrangement 28 comprises a second tube drive unit 30. It is possible, that the first tube movement arrangement 27 and/or the second tube movement arrangement 28 can be operated automatically. Furthermore, the first tube drive unit 29 and/or the second tube drive unit 30 may be self-driven or powered externally, for example by the robotic mechanism 52. The first tube drive unit 29 may be part of the first tube holder 7 and/or the second tube drive unit 30 may be part of the second tube holder 8.

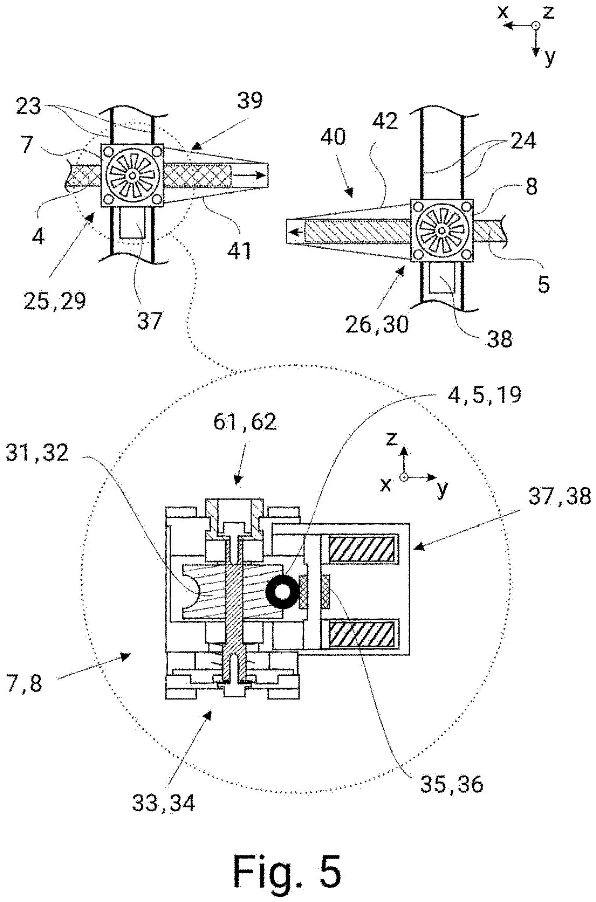

Regarding the design of the tube drive units 29, 30, it is preferred that the first tube drive unit 29 comprises a rotatably mounted first drive roller 31 and/or that the second drive unit 30 comprises a rotatably mounted second drive roller 32. Rotation of the respective drive roller 31, 32 may lead to axial movement, in particular extruding or retracting, of the respective tube 4, 5. As it can be seen exemplarily in Fig. 5 and preferably, the respective tube 4, 5 lies against the respective drive roller 31, 32 such that rotational movement of the drive roller 31, 32 leads to axial movement of the tube 4, 5. The rotational axis of the respective drive roller 31, 32 is vertically arranged.

In a preferred embodiment, the tube drive units 29, 30 are self-driven. Here, the respective drive roller 31, 32 may be rotated as needed and the respective tube 4, 5 may be extruded or retracted accordingly. However, in another also preferred embodiment, the first tube drive unit 29 and/or the second tube drive unit 30 may be powered externally. Here, it is preferred that the first tube drive unit 29 is driven by a first tube drive unit drive 61 and/or the second tube drive unit 30 may be driven by a second tube drive unit drive 62. The first tube drive unit drive 61 and/or the second tube drive unit drive 62 may be designed separately from the respective tube drive unit 29, 30 and may be temporarily connectable to the respective tube drive unit 29, 30 as needed. The first tube drive unit drive 61 and/or the second tube drive unit drive 62 may be transported by the robotic mechanism 52 within the integrated bioprocessing system 1 to the first tube drive unit 29 and/or the second tube drive unit 30. It is particularly preferred that the first tube drive unit drive 61 and/or the second tube drive unit drive 62 can be used to drive several different tube drive units 29, 30, in particular of different cartridges 1d and/or different receptacles 1b. It is possible that the first holder drive unit drive and/or the second holder drive unit drive may be part of the tube connection head 43.

It is preferred that the first tube drive unit 29 comprises a first drive locker 33 for locking the first drive roller 31 such that the first tube 4 is immovable relative to the first tube drive unit 29. Additionally or alternatively, the second tube drive unit 30 comprises a second drive locker 34 for locking the first drive roller 31 such that the second tube 5 is immovable relative to the second tube drive unit 30. The drive lockers 33, 34 might lock the drive rollers 31, 32, after axial arrangement of the tubes 4, 5, as exemplarily shown in Fig. 2a), 2b).

Advantageously, the first tube drive unit 29 comprises a first passive roller 35, which is rotatably mounted and/or the second tube drive unit 30 comprises a second passive roller 36, which is rotatably mounted. For example, as shown in Fig. 5 and preferably, the respective passive roller 35, 36 is arranged relative to the respective drive roller 31, 32 such that the respective tube 4, 5 is arranged in between. The rotational axis of the respective passive roller 35, 36 is vertically arranged.

Furthermore, it is preferred that the first tube drive unit 29 comprises a first pretension arrangement 37 for pretensioning the first passive roller 35 to the first tube 4 and/or that the second tube drive unit 30 comprises a second pretension arrangement 38 for pretensioning the second passive roller 36 to the second tube 5. The respective pretension arrangement 37, 38 enable to increase the grip of the respective drive roller 31, 32 on the respective tube 4, 5, thus, slipping of the respective tube 4, 5 can be prevented. In the exemplarily embodiment of Fig. 5 and preferred, the pretension arrangement 37, 38 comprises at least a spring, which pretensions the respective passive roller 35, 36 in the direction of the respective drive roller 31, 32. The spring might be a compression spring, as in the embodiment according to Fig. 5.

In a further preferred embodiment and as depicted in Fig. 5, the tube arrangement unit 6 comprises a first guiding arrangement 39 for guiding the first tube 4 and/or a second guiding arrangement 40 for guiding the second tube 5. As the tubes 4, 5 might be flexible, the guiding arrangement 39, 40 may improve the accuracy of the respective arrangement of the tubes 4, 5. It is preferred that the first guiding arrangement 39 is part of the first tube holder 7 and/or the second guiding arrangement 40 is part of the second tube holder 8. Particularly preferred is that the first guiding arrangement 39 comprises a first funnel 41 and/or the second guiding arrangement 40 comprises a second funnel 42, as it is exemplarily depicted in Fig. 5. The respective tube 4, 5 may be extruded and/or retracted through the respective funnel 41, 42. The first funnel 41 is preferably arranged on the first tube holder 7 and/or the second funnel 42 is preferably arranged on the second tube holder 8, wherein, further preferably the first funnel 41 is arranged such that the first tube 4 is movable through the first funnel 41 and/or the second funnel 42 is arranged such that the second tube 5 is movable through the second funnel 42. The first funnel 41 and/or the second funnel 42 might be arranged such that the first tube 4 and/or the second tube 5 are being extruded or retracted through the respective funnel 41, 42.

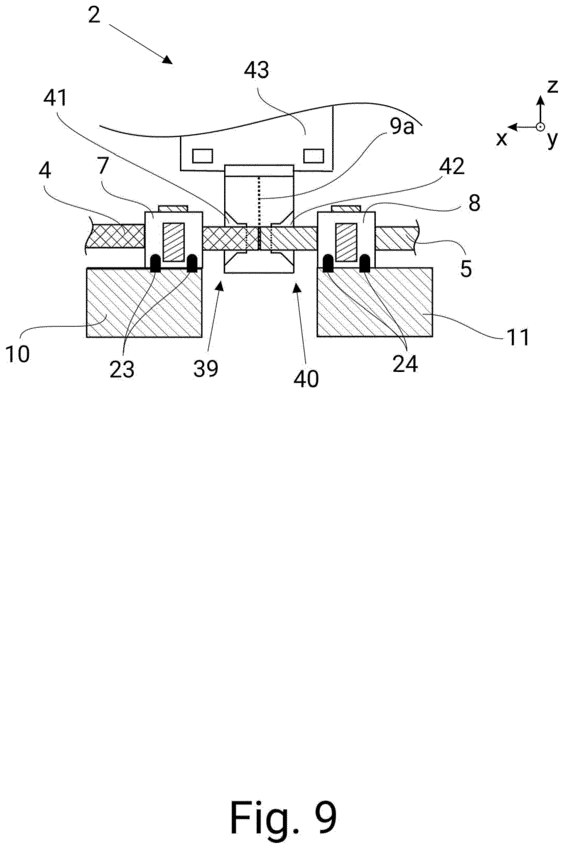

Alternatively, and as schematically depicted in Fig. 9, it is also possible that the first guiding arrangement 39 and the second guiding arrangement 40 are part of a tube connection head 43, in particular of a first tube receiver 58 and a second tube receiver 59. The tube connection head 43 is described in more detail in the following. Particularly preferred is that the first guiding arrangement 39 comprises a first funnel 41 and/or the second guiding arrangement 40 comprises a second funnel 42. The respective tube 4, 5 may be extruded and/or retracted through the respective funnel 41, 42. Here and preferably, the first funnel 41 and the second funnel 42 guide the first tube 4 and the second tube 5 into the tube connection head 43, when the tubes 4, 5 are extruded such that the welding routine can be performed. The first funnel 41 and the second funnel 42 may be arranged on the tube connection head 43, wherein, further preferably the first funnel 41 is arranged such that the first tube 4 is movable through the first funnel 41 and/or the second funnel 42 is arranged such that the second tube 5 is movable through the second funnel 42.

As explained above, each funnel 41, 42 may comprise a sensor to verify that the tube extrusion process was started successfully and additionally to also indicate that the tube was successfully retracted.

If the first guiding arrangement 39 and the second guiding arrangement 40 are part of the tube connection head 43, it is preferred that the first funnel 41 and the second funnel 42 comprise a first funnel opening and a second funnel opening, which extends on the shell surface of the respective funnel 41, 42 along the axis of the respective funnel 41, 42. Via the first funnel opening and the second funnel opening the first tube 4 and the second tube 5 may be removed in cross direction to the funnels 41, 42, in particular after the tube connection 3 is formed and the tubes 4, 5 are connected with each other. Alternatively, the first funnel 41 and the second funnel 42 may be designed respectively as at least two parts such that the first tube 4 and the second tube 5 can be released from the funnels 41, 42, in particular after the tube connection 3 is formed. The first funnel opening and the second funnel opening or the at least two-parted funnels 41, 42 may enable receiving the first tube 4 and the second tube 5, when the tube connection 3 is already formed. This might be beneficial, if the welding routine and the disconnection routine are performed by the tube cutting unit 9 and the tube disconnection arrangement 14 of the tube connection system 2, respectively.

Preferably and shown exemplarily in Fig. 1, the first tube holder 7 is an anchor point 1c, preferably a receptacle anchor point or a cartridge anchor point, and/or the second tube holder 8 is an anchor point 1e, preferably a receptacle anchor point or a cartridge anchor point.

Preferably and shown exemplarily in Fig. 1, the first tube 4 is a transfer tube, preferably a receptacle transfer tube or a cartridge transfer tube, and/or the second tube is a transfer tube, preferably a receptacle transfer tube or a cartridge transfer tube.

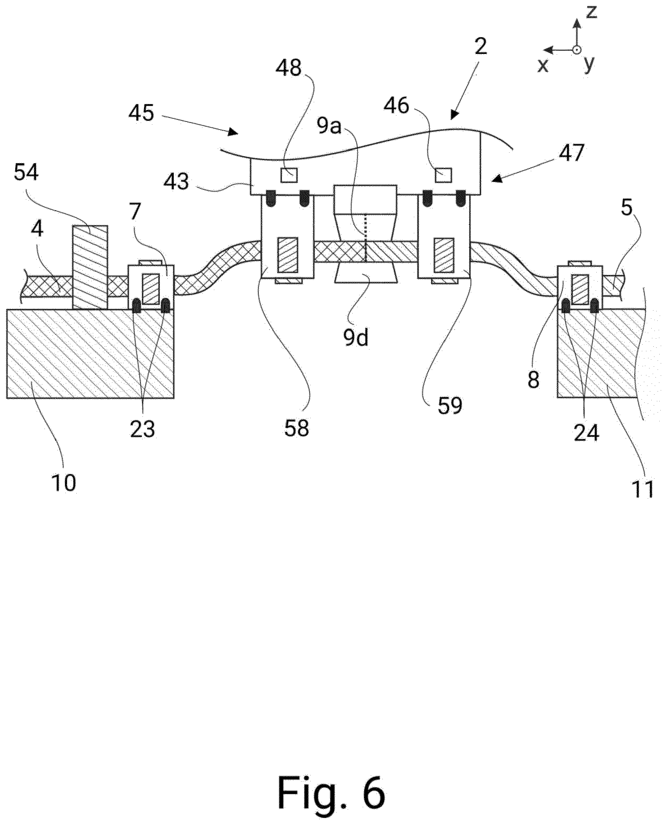

Advantageously, the tube connection system 2 comprises the tube connection head 43, which is preferably unidimensionally, bidimensionally or tridimensionally, movable. Preferably, the tube arrangement unit 6, in particular the first tube holder 7 and/or the second tube holder 8, is designed separately and independently movable from the tube connection head 43, as exemplarily shown in Fig. 3c) and 3d). Alternatively, at least parts of the tube arrangement unit 6, in particular a first tube receiver 58 and/or a second tube receiver 59, might be arranged on the tube connection head 43, as is exemplarily shown in Fig. 6. Here and preferably, the tube receivers 58, 59 are designed as tube holders, with the same or at least partly the same features as the tube holders 7, 8. It is further preferred that the tube cutting unit 9 is designed separately from the tube connection head 43 or at least parts of the tube cutting unit 9 are arranged on the tube connection head 43, as it is for example shown in Fig. 3c) and 6.

Fig. 3c) shows exemplarily a preferred embodiment, in which the tube connection head 43 comprises the tube cutting unit 9. The tube arrangement unit 6, namely the first tube holder 7 and the second tube holder 8, is designed separately from the tube connection head 43. The tube connection head 43 thus can be moved relatively to the tube holders 7, 8. Here, the tube cutting unit 9 might be moved to a certain place in the integrated bioprocessing system 1 to perform the welding routine. Several welding routines may be performed for connection of different tubes 4, 5, for example at different unit operation stations.

Fig. 6 on the other hand shows exemplarily another preferred embodiment, in which the tube connection head 43 comprises the tube cutting unit 9 and at least a part of the tube arrangement unit 6, namely the first tube receiver 58 for receiving a tube, particularly the first tube 4, and the second tube receiver 59 for receiving a tube, particularly the second tube 5. Here, the first tube receiver 58 and the second tube receiver 59 are moveable with the tube connection head 43. The tube connection head 43 may be moved to a certain place in the integrated bioprocessing system 1 to perform the welding routine. Preferably, the welding routine comprises a lacing phase, wherein the tubes 4, 5 are respectively laced or threaded in the tube receivers 58, 59. It is possible that the first tube receiver 58 and/or the second tube receiver 59 are designed such as the tube holders 7, 8. The receiver 58, 59 might be particularly designed as the tube holder 7, 8 described beforehand. Here, the arrangement phase may be performed at least partly by movement of the tube connection head 43.

It is preferred that the tube cutting unit 9 comprises a blade 9a for cutting the first tube 4 and/or the second tube 5, wherein the blade 9a is heatable. Preferably, the tube cutting unit 9 comprises a blade heater 9c for heating the blade 9a and/or a blade mount 9b, wherein the blade 9a is, in particular detachably and/or ejectably, mounted in the blade mount 9b. After the welding routine is performed, the blade 9a might be ejected by the blade mount 9b automatically. It is preferred that the blade 9a and/or the blade heater 9c and/or the blade mount 9b are arranged on the tube connection head 43, as shown exemplarily in Fig. 3c) and 6. It is alternatively also possible that the tube cutting unit 9 comprises alternative cutting elements for cutting the tubes 4, 5, for example a laser or the like. The cutting element may be single-use or multi-use and/or contactless.

For changing the blade 9a, advantageously, the tube connection system 2 comprises a blade compartment 44 for storing several blades 9a, preferably, the blade compartment 44 comprises several blades 9a, which are separately, in particular fully automatically, mountable on the blade mount 9b. Since the blade 9a is preferably detachably, in particular ejectably, mounted in the blade mount 9b, the whole blade changing process, wherein the used blade 9a is changed with an unused blade 9a, might be performed automatically. Here, the used blade 9a, for example after the welding routine is performed, is detached via ejection and the unused blade 9a, which might be located in the blade compartment 44, is mounted.

Alternatively, the blade 9a may be changed by an operator. In this case, the tube cutting unit 9 can be moved to a position of the integrated bioprocessing system 1 that is accessible to an operator.

In a further preferred embodiment, the tube connection system 2 comprises a tube identification arrangement 45 for identifying the first tube 4 held by the first tube holder 7 and/or the second tube 5 held by the second tube holder 8, preferably, the tube identification arrangement 45 is at least partly arranged on the first tube holder 7 and/or is at least partly arranged on the second tube holder 8 and/or is at least partly arranged on the tube connection head 43, further preferably, the tube identification arrangement 45 comprises at least one, in particular optical, identification sensor 46. The tubes 4, 5 to be welded might be identified by the tube identification arrangement 45. In an exemplary embodiment, the means for tube identification may be a color pattern or the like. The welding routine might be performed according to the identified tubes 4, 5.

Preferably, the tube connection system 2 comprises a position determining arrangement 47 for determining the position of the first tube 4 and/or the second tube 5 and/or the first tube holder 7 and/or the second tube holder 8 and/or the tube connection head 43 and/or the first carrier 10 and/or the second carrier 11. Determining the position or positions of the respective components of the tube connection system 2, in particular in the integrated bioprocessing system 1, might be crucial for performing the welding routine, in particular in an automated way. It is in particular preferred that the position of the first tube 4, in particular in the integrated bioprocessing system 1, is determined by determining the position of the first carrier 10 and/or that the position of the second tube 5, in particular in the integrated bioprocessing system 1, is determined by determining the position of the second carrier 11. It is preferred that the position determining arrangement 47 is at least partly arranged on the first tube holder 7 and/or is at least partly arranged on the second tube holder 8 and/or is at least partly arranged on the tube connection head 43 and/or is at least partly arranged on the first carrier 10 and/or the second carrier 11. Further preferably, the position determining arrangement 47 comprises at least one, in particular optical or electrical, position sensor 48 or several, in particular optical and/or electrical, position sensors 48.

For verification reasons, it is preferred in a further embodiment, that the tube connection system 2 comprises a weld verification arrangement 49 for verifying the tube connection 3. The tube connection 3 might be verified by an integrity testing routine. The integrity testing routine might be performed as described before in context with the proposed method. Preferably, the weld verification arrangement 49 comprises one or more tube closing elements 60, such as clamps, for temporarily closing the first tube 4 and/or the second tube 5. The tube closing element 60 or the tube closing elements 60 might be part of the first tube holder 7 and/or the second tube holder 8. It is preferred that the weld verification arrangement 49 comprises at least a, in particular optical or pressure measuring, verification sensor 50 or several, in particular optical and/or pressure measuring, verification sensors 50. The weld verification arrangement 49 preferably comprises at least a tube pressure unit 51 for exerting pressure on the first tube 4 and/or the second tube 5, in particular after welding.

Further preferably, the weld verification arrangement 49 comprises a tube pressure unit 51. The tube pressure unit 51 may comprise one or more, in particular linear, actuators, which compress one of the tubes 4, 5 respectively. A compression at either side of the tube connection 3 is preferred. Preferably, compression is performed by applying pressure to the tubes 4, 5, preferably by the tube pressure unit 51, in particular by compressing the first tube 4 by one of the linear actuators and by compressing the second tube 5 by another of the linear actuators. It is possible that the compression is applied according to a pre-defined compression pattern.

It is preferred that the weld verification arrangement 49 comprises at least a, particularly optical, force or pressure measuring, verification sensor 50 or several, particularly optical, force or pressure measuring, verification sensors 50.

Preferably, the weld verification arrangement 49 comprises at least one verification sensor 50 at either side of the tube connection 3 for verifying the integrity of the tube connection 3. Preferably, at least one verification sensor 50 is designed as a force measuring sensor, wherein the force measuring sensor is used to monitor the force required to compress each tube 4, 5 to a certain degree. Preferably, the force measuring sensor or the force measuring sensors are part of the tube pressure unit 51. In particular, each linear actuator comprises a force measuring sensor. Alternatively or additionally, each tube holder 7, 8 may comprise one force measuring sensor.

Preferably, to verify the integrity of the tube connection 3, the sensor signals obtained over time when compressing each of the tubes 4, 5 are compared to the sensor signals obtained for a tube connection 3 known to be intact and/or to the sensor signals obtained for a tube connection 3 known to be unsafe. Verification of the integrity of the tube connection 3 may for example be performed by analyzing the peak force measured by any of the verification sensors 50, in particular by any of the force measuring sensors, for a given compression. Additionally or alternatively, multivariate data analysis approaches, such as principal component analysis, or machine learning approaches, such as neural networks, may be used to analyze the sensor signals and to determine, if the tube connection 3 is intact or unsafe.

It has been found advantageous if the tube connection system 2 comprises a waste discarding arrangement for discarding waste while cutting the first tube 4 and the second tube 5. Preferably the waste discarding arrangement comprises at least a waste discarding collector and/or at least a waste discarding suction unit. Further preferably the waste discarding collector is movable relative to the first tube holder 7 and/or the second tube holder 8 and/or the waste discarding suction unit is arranged on the tube connection head 43. Alternatively, and preferably, the waste discarding collector can be moved, preferably using the same mechanism that moves the tube connection system 2 such as the robotic mechanism 52, to a location where it can be deposit waste, preferably under automatic control, for subsequent further processing such as incineration.

Furthermore, it is preferred, if the tube connection system 2 comprises a robotic mechanism 52, wherein the tube connection head 43 is attached to the robotic mechanism 52 such that the tube connection head 43 is, preferably, unidimensionally, bidimensionally or tridimensionally, movable. The robotic mechanism 52 might be designed as a robotic arm, such as a multi-axis robot, or as a linear robot, with one, two (also known as x-y-robotic mechanism) or three movement axis (also known as cartesian robotic mechanism). In Fig. 1, for example and preferably, the robotic mechanism 52 is designed as linear robot with three movement axis such that the robotic mechanism 52 can move the tube connection head 43 in three dimensions, namely the x-, y- and z-direction.

Additionally or alternatively, the tube connection system comprises a conveyor mechanism 53, wherein the first carrier 10 and/or the second carrier 11 is unidimensionally, bidimensionally or tridimensionally, movable by the conveyor mechanism 53. Generally, it is also possible, that the robotic mechanism 52 is designed such that the first carrier 10, in particular the receptacle 1b, and/or the second carrier 11, in particular the cartridge 1d, is movable by the robotic mechanism 52 within the integrated bioprocessing system 1. In Fig. 1, for example and possibly, the conveyor mechanism 53 may move the first tube 4 and the second tube 5 by moving the first carrier 10 or rather the receptacle 1b and the second carrier 11 or rather the cartridge 1d respectively in the horizontal plane, which is exemplarily depicted in fig. 1 in x- and y-direction.

In another preferred embodiment, the tube connection system 2 comprises a tube disconnection arrangement 14 for disconnecting the first tube 4 and the second tube 5. Disconnecting of the first tube 4 and the second tube 5 might be performed in a disconnection routine, as described before in context with the proposed method. It is preferred that the tube disconnection arrangement 14 comprises at least a sealing unit 15 and/or a disconnecting unit 17. Further preferably, the tube disconnection arrangement 14 is at least partly arranged on the tube connection head 43. In Fig. 4b) and 4c), for example, the tube disconnection arrangement 14 with the sealing unit 15 and the disconnecting unit 17 are shown.

In this context it is preferred that the sealing unit 15 comprises two sealing surfaces 16, wherein the first tube 4 and/or the second tube 5 and/or the tube connection 3 is or are arrangeable between the sealing surfaces 16 for sealing and wherein the sealing surfaces 16 are movable relative to each other for sealing. It is possible that at least one of the sealing surfaces 16 is heatable. With the sealing unit 15, the sealing phase can be performed. In Fig. 4b), for example, the sealing unit 15 comprises four sealing surfaces 16 or rather two pairs of sealing surfaces 16. Here and preferably, the sealing surfaces 16 are designed respectively as a plane surface, which creates accordingly plane sealings 56, 57. Here and preferably, the plane sealings 56, 57 are transversely orientated to the axis of the tubes 4, 5.

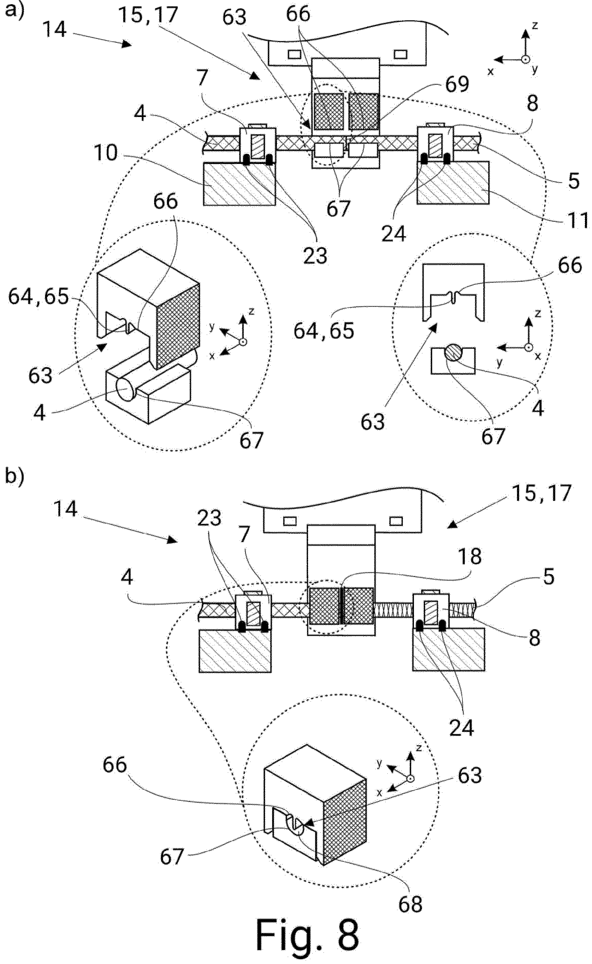

However, it is particularly preferred that the sealing unit 15 comprises a sealing surface 63, which is designed to crimp the first tube 4 and/or the second tube 5 along the axial direction of the respective tube 4, 5 such that the first tube 4 and/or the second tube 5 is or are self-sealed, in particular if the sealing surface 63 is heated when the respective tube 4, 5 is crimped. An example for such an embodiment is shown in Fig. 8a), 8b).

Here and preferably, the sealing surface 63 comprise a crimp element 64, which is designed as a pin 65, in particular as an elongated pin 65. The elongated pin 65 is, as exemplarily shown in Fig. 8a), elongated in axial direction of the tube 4, 5, which is going to be sealed. It is preferred that the sealing surface 63 forms a sealing which in cross section, in particular along the main axis of the sealed tube 4, 5, is U-shaped, as it is depicted exemplarily in Fig. 8b). The cross sections may extend in axial direction of the tube 4, 5 for at least the length of the original diameter of the sealed tube 4, 5, preferably for at least two times the length of the original diameter of the sealed tube 4, 5. It is furthermore possible that the cross section of the sealing comprises at least a subsection over an angular range of at least 180°, preferably at least 235°, wherein within the subsection the original diameter of the sealed tube 4, 5 is maintained. As it can be seen in Fig. 8b) and preferably, the sealing, which is formed by the sealing surface 63, comprises a circumference with at least one inflection point, preferably at least two inflection points.

It is particularly preferred that the sealing surface 63 is a first sealing surface 66 and the sealing unit 15 comprises a second sealing surface 67, which is designed to receive the tube 4, 5 to be sealed. The second sealing surface 67 is designed correspondingly to the tube 4, 5 to be sealed. The second sealing surface 67 particularly comprises a cylindrical groove 68 to receive the respective tube 4, 5. Here and preferably, the sealing unit 15 comprise four sealing surfaces, wherein a pair of sealing surfaces 66, 67 correspond to each other. Each pair of sealing surfaces 67, 66 is arranged at either side of the cutting location 69, where cutting takes place. It is preferred that the crimp element 64 of one of the first sealing surfaces 66 aligns with the center of the groove 68 of the corresponding second sealing surface 67 such that the crimp element 64 compresses the tube 4, 5 in the center and the tube 4, 5 is compressed and sealed without changing the diameter of the tube 4, 5.

Alternatively or additionally, the disconnecting unit 17 comprises a disconnecting laser or a, in particular heatable, disconnecting blade 18, wherein the first tube 4 and/or the second tube 5 and/or the tube connection 3 is or are cuttable by the disconnecting laser or the disconnecting blade 18, preferably in that the disconnecting blade 18 is, in particular detachably and/or ejectably, mounted on the blade mount 9b of the tube connection head 43. With the disconnecting blade 18 or the disconnecting laser, the disconnecting phase can be performed. It is possible that the disconnecting blade 18 is the blade 9a, which is particularly comprised by the tube cutting unit 9. The tube cutting unit 9 may also be the disconnecting unit 17. In general, it is preferably possible that the tube disconnection arrangement 14, in particular the disconnecting unit 17, shares components with the tube connection system 2, in particular the tube cutting unit 9, like the blade 9a, 18, the blade mount 9b and/or the blade compartment 44.

Furthermore, it can be advantageous, if the tube connection system 2 comprises a pumping arrangement 54, wherein the pumping arrangement 54 is designed such that the medium, in particular a fluid, preferably a liquid, can be transferred through the first tube 4 and the second tube 5 after welding and/or such that the medium in the first tube 4 and/or the second tube 5 can be squeezed to minimize dead volume in the first tube 4 and/or the second tube 5. Preferably, the pumping arrangement 54 comprises a peristaltic pump and/or a pressure unit. It is also possible that the pumping arrangement 54 evacuates the first tube 4 and/or the second tube 5 before welding, in particular before the welding phase.

In a preferred embodiment, the tube connection system 2 comprises a data reporting unit 55 for reporting data of welding and/or disconnecting, preferably data, in particular movement data, position data, temperature data, time data and/or pressure data, about the first tube 4 and/or the second tube 5 and/or the tube connection 3 and/or the first tube holder 7 and/or the second tube holder 8 and/or the tube connection head 43 and/or the blade 9a and/or the blade heater 9c and/or the blade mount 9b and/or the blade compartment 44 and/or the robotic mechanism 52 and/or the conveyor mechanism 53 and/or the tube disconnection arrangement 14 and/or the sealing unit 15 and/or the disconnecting unit 17 and/or the end of the first tube 4 and/or the end of the second tube 5.

As a third teaching an integrated bioprocessing system 1 for performing bioprocesses on cell cultures, wherein while performing the bioprocesses a medium used in the bioprocess, in particular the cell culture, is transferable by the integrated bioprocessing system 1 through tubes 4, 5, is provided. The integrated bioprocessing system 1 comprises a first tube 4 for transferring medium and a second tube 5 for transferring medium, wherein the first tube 4 and the second tube 5 are connected by a tube connection 3 and are located at a disconnection location within the integrated bioprocessing system 1. The disconnection location may be a welding location. The integrated bioprocessing system 1 may comprise several disconnection locations.

The first tube 4 is held by a first tube holder 7 and is fluidically connected to a first fluidic structure 12 for receiving and/or providing the medium via the first tube 4. Furthermore, the integrated bioprocessing system 1 comprises a movable first carrier 10, which carries the first tube 4, the first tube holder 7 and the first fluidic structure 12.

The integrated bioprocessing system 1 comprises a tube disconnection arrangement 14 for disconnecting the first tube 4 and the second tube 5 in a disconnection routine at the disconnection location such that the first tube 4 and the second tube 5 are fluidically and/or physically disconnected.

The first carrier 10 and with the first carrier 10 the first tube 4, the first tube holder 7 and the first fluidic structure 12 are transportable from the disconnection location after disconnection of the first tube 4 and the second tube 5.

With the proposed integrated bioprocessing system according to the third teaching, the proposed method can be performed and thus all features and explanations given with regard to the proposed method are fully applicable to the proposed integrated bioprocessing system according to the third teaching and vice versa. Furthermore, all features and explanations given with regard to the proposed integrated bioprocessing system according to the second teaching are fully applicable to the proposed integrated bioprocessing system according to the third teaching and vice versa.

As a fourth teaching a tube disconnection arrangement 14 for disconnecting a first tube 4 and a second tube 5, in particular of an integrated bioprocessing system 1, in a disconnection routine such that the first tube 4 and the second tube 5 are fluidically and/or physically disconnected is provided, wherein the tube disconnection arrangement 14 comprising a sealing unit 15 for sealing the first tube 4 and/or the second tube 5 in a sealing phase such that a first sealing 56 and/or a second sealing 57 is or are formed, wherein the sealing unit 15 comprises at least one sealing surface 63, which is designed to crimp the first tube 4 and/or the second tube 5 along the axial direction of the respective tube 4, 5 such that the first tube 4 and/or the second tube 5 is or are self-sealed.

The proposed tube disconnection arrangement 14 may be used in the proposed method and thus all features and explanations given with regard to the proposed method are fully applicable to the proposed tube disconnection arrangement 14 and vice versa. The proposed tube disconnection arrangement 14 may be used in the proposed integrated bioprocessing system 1 according to the second teaching and/or in the proposed integrated bioprocessing system according to the third teaching and thus all features given with regard to the integrated bioprocessing systems are fully applicable to the proposed tube disconnection arrangement 14 and vice versa.

As a fifth teaching, a sealing unit 15, in particular of a tube disconnection arrangement 14 in an integrated bioprocessing system 1, for sealing a first tube 4 and/or a second tube 5 in a sealing phase such that a sealing 56, 57 is formed, is provided. The sealing unit 15 comprises at least one sealing surface 63, which is designed to crimp the first tube 4 and/or the second tube 5 along the axial direction of the respective tube 4, 5 such that the first tube 4 and/or the second tube 5 is or are self-sealed.

An embodiment of the sealing unit 15 is exemplarily depicted in fig. 8a) and 8b) and is described beforehand in context with the tube disconnection arrangement 14 of the integrated bioprocessing system. All features are thus fully applicable.

The proposed sealing unit 15 may be used in the proposed method and thus all features and explanations given with regard to the proposed method are fully applicable to the proposed sealing unit 15 and vice versa. The proposed sealing unit 15 may be used in the proposed integrated bioprocessing system 1 according to the second teaching and/or in the proposed integrated bioprocessing system according to the third teaching and/or in the tube disconnection arrangement 14 according to the fourth teaching and thus all features given are fully applicable to the proposed sealing unit 15 and vice versa.

As a sixth teaching, a device for transporting a tube 4, 5, in particular within an integrated bioprocessing system 1, wherein the device comprises a carrier 10, 11, a tube 4, 5, a tube holder 7, 8 and a fluidic structure 12, 13, wherein the carrier carries the tube 4, 5, the tube holder 7, 8 and the fluidic structure 12, 13 such that the tube 4, 5, the tube holder 7, 8 and the fluidic structure are transportable with the carrier 10, 11, and, wherein the tube holder 7, 8 holds the tube 4, 5 in a defined position.

The device may be designed as a cartridge 1d or a receptacle 1b. The carrier 10, 11, the tube 4, 5, the tube holder 7,8 and/or the fluidic structure 12, 13 may be designed as described before. All explanations and features given before are fully applicable. Vice versa, all explanations and features regarding the device are fully applicable to the proposed method, the proposed integrated bioprocessing systems 1, the proposed tube disconnection arrangement 14 as well as the proposed sealing unit 15.

Proposed according to a seventh teaching, which is of equal importance, is that the first tube holder 7 is positioned at the welding location by a positioning mechanism separate of the tube connection system 2.

Proposed according to an eight teaching, which is of equal importance, is that the first tube holder 7 fluidically closes, in particular pinches, the first tube 4, in particular reversibly,

Proposed according to a ninth teaching, which is of equal importance, is that the first tube holder 7 extrudes the first tube 4 into the tube connection system 2, in particular prior to any interaction between the first tube 4 and the tube connection system 2.

Proposed according to a tenth teaching, which is of equal importance, is that the first tube holder 7 holds the first tube 4 in a predefined angle or angle range defined with respect to the integrated bioprocessing system 1 as such and/or defined with respect to the tube connection system 2 and/or defined with respect to the second tube 5 prior to any interaction between the first tube 4 and the tube connection system 2. The defined angle for the first and second tube 5 can be seen in fig. 2. Fig. 3 shows how this angle is advantageous for the interaction with the tube connection system 2.

An eleventh teaching is directed to a method of welding, in particular sealing, a tube at least partially along an extension of the tube in particular in an integrated bioprocessing system 1.

## Claims

1. Method for performing bioprocesses on cell cultures, wherein the bioprocesses are being performed on an integrated bioprocessing system (1), wherein while performing the bioprocesses the integrated bioprocessing system (1) transfers a medium used in the bioprocess, in particular the cell cultures, through tubes, wherein the integrated bioprocessing system (1) comprises a tube connection system (2) for connecting tubes of the integrated bioprocessing system (1) as needed by welding, wherein a first tube (4), a first tube holder (7) and a first fluidic structure (12) for receiving and/or providing the medium are carried by a movable first carrier (10), wherein the first tube (4) is held by the first tube holder (7) and is fluidically connected to the first fluidic structure (12), wherein the first carrier (10) and with the first carrier (10) the first tube (4), the first tube holder (7) and the first fluidic structure (12) are transported to a welding location within the integrated bioprocessing system (1), wherein in a welding routine at the welding location the tube connection system (2) connects the first tube (4) and a second tube (5) such that a tube connection (3) is formed, wherein the integrated bioprocessing system (1) transfers the medium from or to the first fluidic structure (12) of the first carrier (10) through the tube connection (3) after welding.

2. Method according to claim 1, **characterized in that** the welding routine comprises an arrangement phase, wherein the first tube (4) and the second tube (5) are being arranged relatively to each other, preferably at least in an axial direction of the first tube (4) and/or the second tube (5), and/or a trimming phase, wherein the first tube (4) and the second tube (5) are being cut, and/or a welding phase, wherein the first tube (4) and the second tube (5) are being brought in contact and merged together such that the tube connection (3) is formed.

3. Method according to claim 1 or 2, **characterized in that** the tube connection system (2) comprises a tube cutting unit (9), that the tube cutting unit (9) comprises a blade (9a) and preferably a blade heater (9c), preferably, that the trimming phase comprises a heating step, that during the heating step the blade heater (9c) heats the blade (9a) to a cutting temperature, and/or, that the trimming phase comprises an alignment step, that the tube cutting unit (9) is aligned relatively to the first tube (4), in particular being in a first cutting position, and/or the second tube (5), in particular being in a second cutting position, and/or, that the trimming phase comprises a cutting step, that the first tube (4), in particular being in the first cutting position, and/or the second tube (5), in particular being in the second cutting position, is or are being cut by the tube cutting unit (9), in particular by the blade (9a).

4. Method according to one of the previous claims, **characterized in that** the tube connection system (2) comprises a tube disconnection arrangement (14), preferably that the tube disconnection arrangement (14) comprises a sealing unit (15) and/or a disconnecting unit (17), further preferably, that the method comprises a disconnection routine, wherein the first tube (4) and the second tube (5) are disconnected, further preferably **in that** the disconnection routine comprises a sealing phase, wherein the first tube (4) and/or the second tube (5) are sealed by the sealing unit (15) such that a first sealing (56) and/or a second sealing (57) are formed, and/or a disconnecting phase, wherein the first tube (4) and the second tube (5) are cut by the tube disconnecting unit (17), in particular between the first sealing (56) and the second sealing (57).

5. Method according to one of the previous claims, **characterized in that** the method comprises temporarily closing the first tube (4) and/or the second tube (5) before the welding routine or before the welding phase is performed, performing an integrity testing routine for verifying the integrity of the tube connection (3) after the welding routine or after the welding phase is performed and depending on the result of the integrity testing routine, if the integrity of the tube connection (3) is verified, reversing the temporary closing of the first tube (4) and/or the second tube (5), and if the integrity of the tube connection (3) is not verified, initiating a emergency routine for preserving the cell cultures.

6. Method according to one of the previous claims, **characterized in that** the method comprises connecting the first tube (4) or a second tube (5) and a third tube (19) of the integrated bioprocessing system (1) by the tube connection system (2) such that a further tube connection (20) is formed, wherein the further tube connection (20) is formed in a welding routine.

7. Integrated bioprocessing system for performing bioprocesses on cell cultures, wherein while performing the bioprocesses a medium used in the bioprocess, in particular the cell cultures, is transferable by the integrated bioprocessing system (1) through tubes, the integrated bioprocessing system (1) comprises a first tube (4) for transferring medium and a second tube (5) for transferring medium, wherein the first tube (4) is held by a first tube holder (7) and is fluidically connected to a first fluidic structure (12) for receiving and/or providing the medium, and a movable first carrier (10), wherein the first tube (4), the first tube holder (7) and the first fluidic structure (12) are carried by the first carrier (10) and wherein the first carrier (10) and with the first carrier (10) the first tube (4), the first tube holder (7) and the first fluidic structure (12) are transportable to a welding location within the integrated bioprocessing system (1), and a tube connection system (2) for connecting the first tube (4) and the second tube (5) in a welding routine at the welding location such that a tube connection (3) is formed by welding, wherein the medium is transferable through the tube connection (3) from or to the first fluidic structure (12) of the first carrier (10) after welding.

8. Integrated bioprocessing system according to claim 7, **characterized in that** the second tube (5) is held by a second tube holder (8) and is fluidically connected to a second fluidic structure (13) for receiving and/or providing the medium, preferably, that the integrated bioprocessing system (1) comprises a movable second carrier (11), wherein the second tube (5), the second tube holder (8) and the second fluidic structure (13) are carried by the second carrier (11) and wherein the second carrier (11) and with the second carrier (11) the second tube (5), the second tube holder (8) and the second fluidic structure (13) are transportable to the welding location.

9. Integrated bioprocessing system according to claim 7 or 8, **characterized in that** the tube connection system (2) comprises a tube arrangement unit (6) for arranging, in an arrangement phase of the welding routine, the first tube (4) and the second tube (5) relatively to each other in at least an axial direction of the first tube (4) and/or the second tube (5), such that the first tube (4) and the second tube (5) can be cut, and a tube cutting unit (9) for cutting the first tube (4) and the second tube (5) in a trimming phase of the welding routine, preferably that the tube arrangement unit (6) is designed such that, in a welding phase of the welding routine, the first tube (4) and the second tube (5) are brought in contact and merged together such that the tube connection (3) is formed.

10. Integrated bioprocessing system according to claim 9, **characterized in that** the first tube holder (7) and/or the second tube holder (8) is or are part of the tube arrangement unit (6), preferably that the tube arrangement unit (6) comprises a first holder movement arrangement (21) for moving the first tube holder (7), in particular orthogonally to the axial direction of the first tube (4), and/or a second holder movement arrangement (22) for moving the second tube holder (8), in particular orthogonally to the axial direction of the second tube (5), more preferably, that the first holder movement arrangement (21) comprises a first holder guide (23) for guiding of the movement of the first tube holder (7) and/or the second holder movement arrangement (22) comprises a second holder guide (24) for guiding the movement of the second tube holder (8), and/or, that the first holder movement arrangement (21) comprises a first holder drive unit (25) and/or the second holder movement arrangement (22) comprises a second holder drive unit (26), preferably that the first holder guide (23) is designed as a first rail unit and/or that the second holder guide (24) is designed as a second rail unit.

11. Integrated bioprocessing system according to one of the claims 9 or 10, **characterized in that** the tube arrangement unit (6), in particular the first tube holder (7), comprises a first tube movement arrangement (27) for moving the first tube (4) held by the first tube holder (7), in particular unidimensionally and/or in an axial direction of the first tube (4), preferably moving the first tube (4) held by the first tube holder (7) relatively to the first tube holder (7), and/or, that the tube arrangement unit (6), in particular the second tube holder (8), comprises a second tube movement arrangement (28) for moving the second tube (5) held by the second tube holder (8) unidimensionally, in particular in an axial direction of the second tube (5), and/or, that the first tube movement arrangement (27) comprises a first tube drive unit (29) and/or the second tube movement arrangement (28) comprises a second tube drive unit (30).

12. Integrated bioprocessing system according to claim 11, **characterized in that** the first tube drive unit (29) comprises a first drive roller (31), which is rotatable, and/or the second drive unit (30) comprises a second drive roller (32), which is rotatable, wherein the first drive roller (31) when rotating moves the first tube (4) in an axial direction of the first tube (4) and/or the second drive roller (32) when rotating moves the second tube (5) in an axial direction of the second tube (5), preferably, that the first tube drive unit (29) comprises a first drive locker (33) for locking the first drive roller (31) such that the first tube (4) is immovable relative to the first tube drive unit (29) and/or the second tube drive unit (30) comprises a second drive locker (34) for locking the first drive roller (31) such that the second tube (5) is immovable relative to the second tube drive unit, (30) and/or, that the first tube drive unit (29) comprises a first passive roller (35), which is rotatable, and/or the second tube drive unit (30) comprises a second passive roller (36), which is rotatable, preferably, that the first tube drive unit (29) comprises a first pretension arrangement (37) for pretensioning the first passive roller (35) to the first tube (4) and/or the second tube drive unit (30) comprises a second pretension arrangement (38) for pretensioning the second passive roller (36) to the second tube (5).

13. Integrated bioprocessing system according to one of the claims 7 to 12, **characterized in that** the tube connection system (2) comprises a, particularly movable, tube connection head (43), preferably that the tube connection head (43) comprises a first tube receiver (58) for receiving a tube, in particular the first tube (4), and/or a second tube receiver (59) for receiving a tube, in particular the second tube (5), further preferably that at least parts of the tube cutting unit (9) are arranged on the tube connection head (43).

14. Integrated bioprocessing system according to one of the claims 7 to 13, **characterized in that** the tube arrangement unit (6) comprises a first guiding arrangement (39) for guiding the first tube (4) and/or a second guiding arrangement (40) for guiding the second tube (5), preferably, that the first guiding arrangement (39) is part of the first tube holder (7) and/or the second guiding arrangement (40) is part of the second tube holder (8), or, that the first guiding arrangement (39) and the second guiding arrangement (40) are part of the tube connection head (43), in particular of the first tube receiver (58) and the second tube receiver (59), more preferably, that the first guiding arrangement (39) comprises a first funnel (41) and/or the second guiding arrangement (40) comprises a second funnel (42), wherein the first funnel (41) is arranged such that the first tube (4) is movable through the first funnel (41) and/or the second funnel (42) is arranged such that the second tube (5) is movable through the second funnel (42).

15. Integrated bioprocessing system according to one of the claims 7 to 14, **characterized in that** the tube connection system (2) comprises a position determining arrangement (47) for determining the position of the first tube (4) and/or the second tube (5) and/or the first tube holder (7) and/or the second tube holder (8) and/or the tube connection head (43) and/or the first carrier (10) and/or the second carrier (11), preferably that the position determining arrangement (47) comprises at least one, in particular optical or electrical, position sensor (48) or several, in particular optical and/or electrical, position sensors (48), which are at least partly arranged on the first tube holder (7) and/or the second tube holder (8) and/or the tube connection head (43) and/or the first carrier (10) and/or the second carrier (11).

16. Integrated bioprocessing system according to one of the claims 7 to 15, **characterized in that** the tube connection system (2) comprises a robotic mechanism (52), in particular an x-y robotic mechanism or a cartesian robotic mechanism, wherein the tube connection head (43) is attached to the robotic mechanism (52) such that the tube connection head (43) is at least unidimensional, preferably bidimensional or tridimensional, movable, and/or, that the tube connection system (2) comprises a conveyor mechanism (53), wherein the first carrier (10) and the first tube holder (7) and/or the second carrier (11) and the second tube holder (8) are at least unidimensional, preferably bidimensional or tridimensional, movable, by the conveyor mechanism (53).

17. Integrated bioprocessing system, in particular according to one of claims 7 to 16, for performing bioprocesses on cell cultures, wherein while performing the bioprocesses a medium used in the bioprocess, in particular the cell culture, is transferable by the integrated bioprocessing system (1) through tubes, the integrated bioprocessing system (1) comprises a first tube (4) for transferring medium and a second tube (5) for transferring medium, wherein the first tube (4) and the second tube (5) are connected by a tube connection (3) and are located at a disconnection location within the integrated bioprocessing system (1), wherein the first tube (4) is held by a first tube holder (7) and is fluidically connected to a first fluidic structure (12) for receiving and/or providing the medium via the first tube (4), and a movable first carrier (10), wherein the first tube (4), the first tube holder (7) and the first fluidic structure (12) are carried by the first carrier (10), and a tube disconnection arrangement (14) for disconnecting the first tube (4) and the second tube (5) in a disconnection routine at the disconnection location such that the first tube (4) and the second tube (5) are fluidically and/or physically disconnected, wherein the first carrier (10) and with the first carrier (10) the first tube (4), the first tube holder (7) and the first fluidic structure (12) are transportable from the disconnection location after disconnection of the first tube (4) and the second tube (5).

18. Sealing unit, in particular of a tube disconnection arrangement (14) in an integrated bioprocessing system (1), for sealing a first tube (4) and/or a second tube (5) in a sealing phase such that a sealing (56, 57) is formed, wherein the sealing unit (15) comprises at least one sealing surface (63), which is designed to crimp the first tube (4) and/or the second tube (5) along the axial direction of the respective tube (4, 5) such that the first tube (4) and/or the second tube (5) is or are self-sealed.

19. Device for transporting a tube (4, 5), in particular within an integrated bioprocessing system (1) according to claims 7 to 16, wherein the device comprises a carrier (10, 11), a tube (4, 5), a tube holder (7, 8) and a fluidic structure (12, 13), wherein the carrier (10, 11) carries the tube (4, 5), the tube holder (7, 8) and the fluidic structure (12, 13) such that the tube (4, 5), the tube holder (7, 8) and the fluidic structure (12, 13) are transportable with the carrier (10, 11), and, wherein the tube holder (7, 8) holds the tube (4, 5) in a defined position.
